(19) **Europäisches Patentamt / European Patent Office / Office européen des brevets**

(11) **EP 1 671 128 B1**

(12) **EUROPEAN PATENT SPECIFICATION**

(45) Date of publication and mention of the grant of the patent:
**17.02.2010 Bulletin 2010/07**

(21) Application number: **04809691.1**

(22) Date of filing: **08.09.2004**

(51) Int Cl.:
*G01N 33/58* (2006.01)   *G01N 33/542* (2006.01)

(86) International application number:
**PCT/US2004/029099**

(87) International publication number:
**WO 2005/026730 (24.03.2005 Gazette 2005/12)**

(54) **Applications of the resonance energy transfer between terbium and GFP**

Anwendungen des Resonanzenergietransfers zwischen Terbium und GFP

Applications du transfert d'énergie par résonance entre terbium et GFP

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **12.09.2003 US 502377 P**

(43) Date of publication of application:
**21.06.2006 Bulletin 2006/25**

(73) Proprietor: **Life Technologies Corporation Carlsbad, CA 92008 (US)**

(72) Inventor: **VOGEL, Kurt Madison, WI 53717 (US)**

(74) Representative: **Couchman, Jonathan Hugh et al Harrison Goddard Foote Belgrave Hall Belgrave Street Leeds LS2 8DD (GB)**

(56) References cited:
**US-A1- 2003 059 811**

• **HEMMILA I ET AL: "Time-resolved fluorometry: An overview of the labels and core technologies for drug screening applications" DRUG DISCOVERY TODAY 1997 UNITED KINGDOM, vol. 2, no. 9, 1997, pages 373-381, XP002338475 ISSN: 1359-6446**

• **ZAMAN G J R ET AL: "Fluorescence assays for high-throughput screening of protein kinases." COMBINATORIAL CHEMISTRY AND HIGH THROUGHPUT SCREENING, vol. 6, no. 4, June 2003 (2003-06), pages 313-320, XP008050422 ISSN: 1386-2073 & SEETHALA RAMAKRISHNA ET AL: "A homogeneous, fluorescence polarization assay for Src-family tyrosine kinases" ANALYTICAL BIOCHEMISTRY, ACADEMIC PRESS, SAN DIEGO, CA, US, vol. 253, 15 November 1997 (1997-11-15), pages 210-218, XP002150325 ISSN: 0003-2697**

• **SILLS M A ET AL: "COMPARISON OF ASSAY TECHNOLOGIES FOR A TYROSINE KINASE ASSAY GENERATES DIFFERENT RESULTS IN HIGH THROUGHPUT SCREENING" JOURNAL OF BIOMOLECULAR SCREENING, LARCHMONT, NY, US, vol. 7, no. 3, 2002, pages 191-214, XP009024655 ISSN: 1087-0571**

• **LOWERY R G ET AL: "Fluorescence methods for dissecting steroid hormone signal transduction" INTERNET ARTICLE, [Online] February 2002 (2002-02), page 1, XP002338476 Retrieved from the Internet: URL:http://www.invitrogen.com/downloads/L0 745.pdf> [retrieved on 2005-07-29]**

• **ZHANG Y ET AL: "Optimization of measurement parameters and performance comparison for analyst(TM) GT and analyst HT or acquest" JALA - JOURNAL OF THE ASSOCIATION FOR LABORATORY AUTOMATION 2003 UNITED STATES, vol. 8, no. 2, April 2003 (2003-04), pages 71-73, XP002338474 ISSN: 1535-5535**

EP 1 671 128 B1

• FOWLER A ET AL: "A multi-Modality Assay Platform for Ultra-High Throughput Screening" CURRENT PHARMACEUTICAL BIOTECHNOLOGY, BENTHAM SCIENCE PUBLISHERS, BOCA RATON,FL, US, vol. 1, no. 3, 2000, pages 265-281, XP002327453 ISSN: 1389-2010

• FRONCZAK J A ET AL: "Rapid Conversion of FP to TR-FRET Assays using Terbium-based LanthaScreenTM Technology" INTERNET ARTICLE, [Online] 16 September 2003 (2003-09-16), page 1, XP002338477 Retrieved from the Internet: URL:http: //www.invitrogen.com/downloads/76 2-038072.pdf> [retrieved on 2005-07-29]

• VEDVIK K L AT AL: "Increasing Information Content Using Multimode Fluorescent Assays" INTERNET ARTICLE, [Online] 16 September 2003 (2003-09-16), page 1, XP002338478 Retrieved from the Internet: URL:http: //www.invitrogen.com/downloads/76 2-038071.pdf> [retrieved on 2005-07-29]

• SELVIN P R: "Principles and biophysical applications of lanthanide-based probes" ANNU REV BIOPHS BIOMOL STRUCT, vol. 31, 2002, pages 275-302, XP008060956

**Description**

**TECHNICAL FIELD**

This invention relates to assays employing fluorescence polarization (FP) and/or time-resolved resonance energy transfer (TR-RET) detection methods, and more particularly to methods for monitoring and measuring molecular interactions, such as competitive binding or enzymatic activity events, using the same.

**BACKGROUND**

Drug discovery can involve the systematic and/or high-throughput screening of diverse chemical libraries containing thousands of members. The size and complexity of these libraries, when coupled with the expense and length of the FDA approval process, have resulted in the need for simple, efficient, and homogeneous assays for probing molecular interactions.

Luminescence-based techniques, including fluorescence polarization (FP), resonance energy transfer (RET), and luminescence resonance energy transfer methods (LRET) methods, are typically highly sensitive, homogenous methods for probing molecular interactions. Background luminescence (e.g., fluorescence or luminescence from assay components), non-specific interactions of assay components, and light scattering, however, can limit the sensitivity of luminescence-based assays, particularly when luminophores having short lifetimes are used, resulting in the detection of false positives or false negatives in a drug screen. Follow-up screening of individually-picked compounds or the use of multiple screens may be required to validate screen results. It would be useful to have screening methodologies that could increase the information content of fluorescent or luminescent assays and reduce the number of spurious results encountered in drug screens.

Hemmila I et al (1997) Drug Discovery Today, 2(9) 373-381 teaches that time-resolved fluoremetry (TRF) with lanthanide chelate labels is a well-established technology in diagnostics. Europium and terbium are mentioned as examples of suitable lanthanides It is described that one TRF is based on europium cryptate as donor and a phycobiliprotein (derivative) as acceptor, and that rhodamine is an acceptor for terbium.

Zhang Y et al (2003) J. Assoc. Lab. Autom., 8(2), 71-73 describes a plate reader supporting *inter alia* TRF. An example uses europium solution in TRF.

Zaman G J R et al (2003) Combinat. Chem. High Throughput Screen, 6(4), 3113-320 relates to fluorescence assays for screening protein kinases. It is reported that probes were developed based on chelates of lanthanide ions, such as europium and terbium, it is further reported that TR-FRET (time-resolved fluorescence resonance energy transfer) has been realised by the binding of a europium-labelled anti-phospho antibody to a peptide substrate labelled with allophycocyanin (APC), the APC acting as an acceptor probe.

Selvin PR (2002) Annu. Rev. Biophs. Biomol. Struct., 31, 275-302 teaches that Tb(III) and GFP represent a good luminescence resonance energy transfer (LRET) pair.

**SUMMARY**

The invention relates to the monitoring of molecular interactions, particularly enzymatic activities.

In a first aspect, there is provided an article of manufacture comprising:

a) packaging material;
b) a first binding partner comprising a Tb(III) metal complex ; and
c) a second binding partner comprising a GFP, wherein said second binding partner specifically binds said first binding partner,

and wherein one of the first and second binding partners has binding specificity for either the product or substrate of an enzymatic activity.

The articles of manufacture are useful if a second aspect of the invention, namely a method for forming a test sample, comprising:

contacting an enzyme with a substrate for the enzyme under conditions effective for an enzymatic activity of the enzyme to form a product from the substrate, the contacting being carried out in the presence of a potential Modulator of the enzymatic activity, and
forming a test sample by either

i) contacting the mixture of the enzyme, substrate and potential modulator with (a) a binding partner having specificity for either the substrate or a product of enzymatic activity on the substrate, and (b) a luminescent tracer capable of binding with the binding partner, wherein one of the binding partner and the tracer includes a luminescent Tb(III)-containing metal complex whilst the other includes a GFP; or

ii) contacting the mixture of the enzyme, substrate and potential modulator with (a) a binding partner having specificity for either the substrate or a product of enzymatic activity on the substrate, and (b) a tracer capable of binding with the binding partner, wherein one of the binding partner and the substrate includes a luminescent Tb(III)-containing metal complex whilst the other includes a GFP.

In a further step, a test sample is exposed to polarized light and the polarization of fluorescent emission from the test sample is measured. The test sample can also be exposed to light having a wavelength in the range of 250 nm to 750 nm and the fluorescence emission of the test sample can be measured. The exposure to polarized light and the measurement of polarization of fluorescent emission can be performed prior to, simultaneously with, or after the exposure to light having a wavelength in the range from 250 nm to 750 nm and the measurement of fluorescence emission. The test compound is identified as affecting binding between the first binding partner and the second binding partner when the fluorescence polarization measurement or the fluorescence emission measurement, or both, of the test sample is different from the fluorescence polarization measurement or the fluorescence emission measurement of a corresponding control sample lacking the test compound.

A Tb(III) metal complex can include an organic antenna moiety, a metal liganding moiety and a Tb(III) metal ion. An organic antenna moiety can be selected from the group consisting of: rhodamine 560, fluorescein 575, fluorescein 590, 2-quinolone, 4-quinolone, 4-trifluoromethylcoumarin (TFC), 7-diethyt-amino-coumarin-3-carbohydrazide, 7-amino-4-methyl-2-coumarin (carbostyril 124), 7-amino-4-methyl-2-coumarin (coumarin 120), 7-amino-4-trifluoromethyl-2-coumarin (coumarin 124), and aminomethyltrimethylpsoralen. A metal liganding moiety can be a metal chelating moiety selected from the group consisting of: EDTA, DTPA, TTHA, DOTA, NTA, HDTA, DTPP, EDTP, HDTP, NTP, DOTP, DO3A, DOTAGA, and NOTA.

In some embodiments, a Tb(III) metal complex has a structure:

$$-L_n-A-S_n-C_M,$$

or

$-L_n-C_M-S_n-A$, where A represents an organic antenna moiety; L represents a linker; S represents a spacer; n can be 0 or 1; C represents a metal chelating moiety; and M represents a Tb(III) metal ion coordinated to C.

In another aspect, the invention provides a use of an article of the present invention in performing monitoring by one or more fluorescent techniques of an interaction of first and second binding partners, wherein the first binding partner comprises a Tb(III)-containlng metal complex and the second binding partner comprises a GFP, and one of the first and second binding partners has binding specificity for either the product or substrate of an enzymatic activity.

The articles of the invention are useful in a method for identifying a modulator of an enzymatic activity. The method includes contacting an enzyme with a substrate for the enzyme, where the contacting is carried out under conditions effective for an enzymatic activity of the enzyme to form a product from the substrate and where the contacting is carried out in the presence of a potential modulator of the enzymatic activity. The enzyme, substrate, and potential modulator are then contacted with a first binding partner and a tracer to form a test sample. The first binding partner has binding specificity for either the product or the substrate of the enzymatic activity. The first binding partner is capable of binding the tracer.

The tracer can be unlabeled or it can include a luminescent Tb(III)-containing metal complex or a fluorescent GFP acceptor moiety, e.g., a "luminescent tracer." For example, in one embodiment of the method, one of a first binding partner and a tracer includes a said luminescent metal complex, while the other includes a said fluorescent acceptor moiety. In other embodiments, one of a first binding partner and a substrate includes a luminescent metal complex, while the other includes a fluorescent acceptor moiety.

A test sample is then exposed to polarized light and the polarization of fluorescent emission from the test sample is measured. The test sample can also be exposed to light having a wavelength in the range from 250 nm to 750 nm and the fluorescence emission from the test sample is measured. The exposure to polarized light and the measurement of polarization of fluorescent emission can be performed prior to, simultaneously with, or after the exposure to light having a wavelength in the range from 250 nm to 750 nm and the measurement of fluorescence emission.

A potential modulator is identified as a modulator of the enzymatic activity when the fluorescence polarization measurement or the fluorescence emission measurement, or both, of the test sample is different from the fluorescence polarization measurement or fluorescence emission measurement, respectively, of a corresponding control sample lacking the potential modulator. In any of the methods described herein, a difference in the fluorescence polarization measurement of a test sample as compared to a control sample can be from about 30 mP to about 450 mP. A difference in fluorescence polarization measurement of a test sample as compared to a control sample can also be from about a 10% to about a 10,000% increase or decrease. The fluorescence emission of a test sample or a control sample can be measured at two or more wavelengths. A ratio of fluorescence emission measurements of a test sample or a control sample at two wavelengths can also be calculated.

An enzymatic activity can be selected from the group consisting of kinase activity, phosphatase activity, glucuronidase activity, prenylation, glycosylation, methylation, demethylation, acylation, acetylation, ubiquitination, sulfation, proteolysis, nuclease activity, nucleic acid polymerase activity, nucleic acid reverse transcriptase activity, nucleotidyl transferase activity, and polynucleotide translation activity.

The invention also provides articles of manufacture. An article of manufacture, such as a kit, can include packaging material; and a first binding partner and a second binding partner, where the second binding partner is capable of binding the first binding partner. One binding partner comprises a luminescent Tb(III)-containing metal complex and the other a GFP fluorescent acceptor moiety.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

## DESCRIPTION OF DRAWINGS

FIG. 1 is a schematic indicating one embodiment of a multiplex FP/TR-RET assay.

FIG. 2 and FIG. 3 demonstrate the chemical structure of two luminescent metal chelates comprising organic antenna moieties.

FIG. 4 demonstrates the normalized excitation/emission spectrum for a terbium chelate comprising an organic antenna moiety (CS124).

FIG. 5 demonstrates the relationship, at various fluorophore lifetimes, between expected polarization values (mP) and the MW of a complex between a first and second binding partner.

FIG. 6 demonstrates a direct binding assay using a Tb-chelate labeled antibody and a fluorescein-labeled phosphopeptide tracer.

FIG. 7 demonstrates a competition assay measured with FP and TR-RET between a complex of a Tb-chelate labeled antibody and a fluorescein-labeled phosphopeptide tracer titrated with an unlabeled phosphopeptide competitor.

FIG. 8A and FIG. 8B demonstrate the results of screening a chemical library using both FP and TR-RET modes.

FIG. 9A and FIG. 9B demonstrate the results of measuring the interaction between an anti-phosphorylated CREB antibody and four different phosphorylated peptide tracers using FP and TR-RET measurements, respectively.

FIG. 10A and FIG. 10B demonstrate a titration of PKA enzyme measured by TR-RET and the Z'-factor for the assay, respectively.

FIG. 11A and FIG. 11B demonstrate comparable EC50 values obtained using FP and TR-RET to measure the interaction between Estrogen Receptor β and Estradiol.

FIG. 12 demonstrates the absorbance profile of a chelate and a chelate-antibody conjugate.

FIG. 13 demonstrates the polarization measurement versus antibody concentration at varying chelate:Ab ratios.

FIG. 14 demonstrates the normalized signal (FP or IR-RET) for a library screen.

FIG. 15 is a plot of normalized FP data vs. normalized TR-RET data for a library screen.

FIG. 16 is a plot of FP and TR-RET titration data obtained for two inhibitor compounds identified in a library screen.

FIG. 17 demonstrates that the TR-RET detection mode is resistant to background fluorescence signals.

FIG. 18 represents a re-analysis of spurious data obtained in a library screen, demonstrating the resistance of the TR-RET mode to light scattering.

FIG. 19 represents a re-analysis of spurious data obtained in a library screen.

FIG. 20A and 20B demonstrate the comparable EC50 values obtained using FP and TR-RET to measure the interaction between an antibody and a phosphopeptide.

FIG. 21A and 21B demonstrate the Z'-factors obtained in TR-RET mode and FP mode for the interaction of an antibody and a phosphopeptide.

FIG. 22A and 22B demonstrate FP and TR-RET data obtained for a competition assay between a complex of a Tb-chelate-labeled anti-histag antibody and a labeled histag tracer titrated with an unlabeled histag-labeled protein competitor.

Like reference symbols in the various drawings indicate like elements.

## DETAILED DESCRIPTION

The invention is based on the discovery that the use of multiple detection modes (multiplex modes) can improve the sensitivity, reliability, and information content of assays that probe a wide variety of molecular interactions, including competitive binding events and enzymatic activities (e.g., post-translational modifications). Methods of the invention allow the use of both fluorescence polarization (FP) and time-resolved resonance energy transfer (TR-RET) detection modes. Use of the multiplex methods minimizes the number of false positive and false negative hits in screening assays, resulting in increased confidence in the integrity of screening results. In addition, use of the multiplex methods facilitates re-analysis of potentially spurious results due to background fluorescence interference and light scattering. Screening assays based on FP can be easily converted to TR-RET or multiplex FP/IR-RET assays using the methods of the present invention, allowing the probing of molecular interactions in either or both fluorescent modes. Compositions suitable for use in the presently described methods are also described, including mixtures of compositions.

### *Definitions*

Generally, the nomenclature used herein and many of the fluorescence, luminescence, computer, detection, chemistry, and laboratory procedures described herein are commonly employed in the art. Standard techniques are generally used for chemical synthesis, fluorescence or luminescence monitoring and detection, optics, molecular biology, and computer software and integration. Chemical reactions, cell assays, and enzymatic reactions are typically performed according to the manufacturer's specifications where appropriate. See, generally, Lakowicz, J.R. Topics in Fluorescence Spectroscopy, (3 volumes) New York: Plenum Press (1991), and Lakowicz, J. R. Emerging applications of fluorescence spectroscopy to cellular imaging: lifetime imaging, metal-ligand probes, multi photon excitation and light quenching, Scanning Microsc. Suppl. Vol. 10 (1996) pages 213-24, for fluorescence techniques; Sambrook et al., Molecular Cloning: A Laboratory Manual, 2ed. (1989) Cold Spring Harbor Laboratory Press, Cold Spring Harbor, N.Y., for molecular biology methods; Cells: A Laboratory Manual 1st edition (1998) Cold Spring Harbor Laboratory Press, Cold Spring Harbor, N.Y., for cell biology methods; and *Optics Guide 5* Melles Griot® Irvine CA, and Optical Waveguide Theory, Snyder & Love (published by Chapman & Hall) for general optical methods.

General methods for performing a variety of fluorescent or luminescent assays on luminescent materials are known in the art and are described in, e.g., Lakowicz, J.R., Topics in Fluorescence Spectroscopy, volumes 1 to 3, New York: Plenum Press (1991); Herman, B., Resonance Energy Transfer Microscopy, in Fluorescence Microscopy of Living Cells in Culture, Part B, Methods in Cell Biology, vol. 30, ed. Taylor, D.L. & Wang, Y.-L., San Diego: Academic Press (1989), pp. 219-243; Turro, N.J., Modem Molecular Photochemistry, Menlo Park: Benjamin/Cummings Publishing Col, Inc. (1978), pp. 296-361; and Bernard Valeur, "Molecular Fluorescence: Principles and Applications" Wiley VCH, 2002. Guidance in the selection and use of specific resonance acceptor moieties is available at, for example, Berlman, I.B., Energy transfer parameters of aromatic compounds, Academic Press, New York and London (1973), which contains tables of spectral overlap integrals for the selection of resonance energy transfer pairs. Additional information sources include the Molecular Probes Catalog (2003) and website; and Tsien et al., 1990 Handbook of Biological Confocal Microscopy, pp. 169-178. Instruments useful for performing FP and/or RET and TR-RET applications are available from Tecan Group Ltd. (Switzerland) (Ultra, Ultra 384, Ultra Evolution); Perkin-Elmer (Boston, MA) (Fusion, EnVision, Victor V, and ViewLux), Amersham Bioscience (Piscataway, NJ) (LeadSeeker); and Molecular Devices Corporation (Sunnyvale, CA) (Analyst AD, GT, and HT).

Commonly used chemical abbreviations that are not explicitly defined in this disclosure may be found in The American Chemical Society Style Guide, Second Edition; American Chemical Society, Washington, DC (1997), "2001 Guidelines for Authors" J. Org. Chem. 66(1), 24A (2001), and "A Short Guide to Abbreviations and Their Use in Peptide Science" J. Peptide. Sci. 5, 465-471 (1999).

Abbreviations: t-Boc, tert-butyloxycarbonyl; Bzl, benzyl; PTK, protein tyrosine kinase; Fmoc, fluorenylmethyloxycarbonyl; ELISA, enzyme-linked immuno absorbant assay; FP, fluorescence polarization; FITC, fluorescein isothiocyanate; RET, resonance energy transfer; FRET, fluorescence resonance energy transfer or Forster resonance energy transfer; TR, time resolved; FAM, carboxyfluorescein.

As employed throughout the disclosure, the following terms, unless otherwise indicated, shall be understood to have the following meanings:

The terms "antibody" and "antibodies" include polyclonal antibodies, monoclonal antibodies, humanized or chimeric antibodies, single chain Fv antibody fragments, Fab fragments, and $F(ab)_2$ fragments. Polyclonal antibodies are heterogeneous populations of antibody molecules that are specific for a particular antigen, while monoclonal antibodies are homogeneous populations of antibodies to a particular epitope contained within an antigen. A chimeric antibody is a molecule in which different portions are derived from different animal species, such as those having a variable region derived from a mouse monoclonal antibody and a human immunoglobulin constant region. The term "epitope" refers to an antigenic determinant on an antigen to which an antibody binds. Epitopes usually consist of chemically active surface groupings of molecules such as amino acids, sugar side chains, or chemical moieties (e.g., from organic compounds) and typically have specific three-dimensional structural characteristics as well as specific charge characteristics. Epitopes can consist of a series of contiguous amino acids, e.g., 5 contiguous amino acids. In other embodiments, an epitope can be a discontinuous epitope, e.g., the epitope is a particular arrangement of amino acids in space that results from the secondary, tertiary, and/or quaternary folding of a polypeptide. In yet other embodiments, an epitope can consist of a modified amino acid side chain, e.g., a phosphorylated tyrosine, serine, or threonine. Monoclonal antibodies are particularly useful in the present invention.

The term "RET" means resonance energy transfer, and refers to the radiationless transmission of an energy quantum from its site of absorption (the donor) to the site of its utilization (the acceptor) in a molecule, or system of molecules, by resonance interaction between donor and acceptor species, over distances considerably greater than interatomic, without substantial conversion to thermal energy, and without the donor and acceptor coming into kinetic collision. A donor is a moiety that initially absorbs energy (e.g., optical energy or electronic energy). A luminescent metal complex as described herein can comprise two donors: 1) an organic antenna moiety, which absorbs optical energy (e.g., from a photon); and 2) a Tb(III) metal ion, which absorbs electronic energy (e.g., transferred from an organic antenna moiety). RET is sometimes referred to as fluorescent resonance energy transfer or Forster resonance energy transfer (both abbreviated FRET).

The term "acceptor" refers to a chemical or biological moiety that accepts energy via resonance energy transfer. In RET applications, acceptors may re-emit energy transferred from a donor fluorescent or luminescent moiety as fluorescence (e.g., RET or TR-RET) and are "fluorescent acceptor moieties." As used herein, such a donor fluorescent or luminescent moiety and an acceptor fluorescent moiety are referred to as a "RET pair." The acceptors used in the invention are GFP and GFP derivatives. Acceptors, including fluorescent acceptor moieties, can also be useful as fluorescent probes in FP assays.

The terms "label" or labeled" refer to the inclusion of a luminescent metal complex or a fluorescent acceptor moiety on a first binding partner, second binding partner, tracer, test compound, potential modulator, substrate, or product, as described herein.

The term "modulates" refers to partial or complete enhancement or inhibition of an activity or process (e.g., by attenuation of rate or efficiency).

The term "modulator" refers to a chemical compound (naturally occurring or non-naturally occurring), such as a biological macromolecule (e.g., polynucleotide, polypeptide, hormone, polysaccharide, lipid), an organic molecule (e.g., a small organic molecule), or an extract made from biological materials such as bacteria, plants, fungi, or animal (particularly mammalian, including human) cells or tissues. Modulators may be evaluated for potential activity as inhibitors or enhancers (directly or indirectly) of a biological process or processes (e.g., agonist, partial antagonist, partial agonist, inverse agonist, antagonist, antineoplastic agents, cytotoxic agents, inhibitors of neoplastic transformation or cell proliferation, cell proliferation-promoting agents, and the like) by inclusion in screening assays described herein. The activity of a modulator may be known, unknown, or partially known.

The term "non-naturally occurring" refers to the fact that an object, compound, or chemical cannot be found in nature. For example, a polypeptide or polynucleotide that is present in an organism (including viruses) that can be isolated from a source in nature and which has not been intentionally modified by man in the laboratory is naturally-occurring, while such a polypeptide or polynucleotide that has been intentionally modified by man is non-naturally occurring.

The term "organic molecule" refers to compounds having a molecular skeleton containing a covalent arrangement of one or more of the elements C, N, H, O, S, and P, and typically having a molecular weight less than 10000 Daltons. Organic molecules having a molecular weight less than 5000 Daltons may be referred to as "small organic molecules."

The term "polypeptide" refers to a polymer of two or more amino acids joined together through amide bonds. A polypeptide can be an entire protein (e.g., isolated from a natural source or an expression system), a fragment of a protein, an enzymatically or chemically synthesized and/or modified version of a protein or protein fragment, or an amino acid sequence designed *de novo* (e.g., not based on a known protein sequence). Polypeptides can be 2-1000 amino acids in length (e.g., 2-900, 2-800, 2-700, 2-600, 2-500, 2-480, 2-450, 2-300, 2-200, 2-100, 2-50, 2-25, 5-900, 5-800, 5-700, 5-600, 5-500, 5-450, 5-300, 5-200, 5-100, 5-50, 5-25, 10-900, 10-800, 10-700, 10-600, 10-500, 10-450, 10-300, 10-200, 10-100, 10-50, 20-900, 20-800, 20-700, 20-600, 20-500, 20-450, 20-300, 20-200, 20-100, or 20-50 amino acids in length). Amino acids may be natural or unnatural amino acids, including, for example, beta-alanine, phenylglycine, and homoarginine. For a review, see Spatola, A.F., in Chemistry and Biochemistry of Amino Acids, Peptides and Proteins, B. Weinstein, eds., Marcel Dekker, New York, p. 267 (1983). All of the amino acids used in the present invention may be either the D- or L-isomer. Particularly useful chemically modified or substituted amino acids including phosphorylated (e.g., phospho-serine (phosphorylated at the hydroxyl of the side chain), phospho-tyrosine (phosphorylated at the OH of the side-chain phenyl ring), and phospho-threonine (phosphorylated at the hydroxyl of the size chain)), sulfated, methylated, or prenylated amino acids.

The terms "post-translational modification" and "post-translational type modification" are used interchangeably and refer to enzymatic or non-enzymatic modification of one or more amino acid residues in a polypeptide. Typical modifications include phosphorylation, dephosphorylation, glycosylation, methylation, sulfation, ubiquitination, acylation, acetylation, prenylation, and ADP-ribosoylation. Preferred post-translational type modifications include phosphorylation and dephosphorylation. The term post-translational modification includes non-covalent modifications that may affect polypeptide activity (e.g., protein activity), structure, or function, such as polypeptide-polypeptide interactions or the binding of ligands, allosteric modulators, other modulators, or second messengers such as calcium, cAMP, or inositol phosphates.

The term "test compound" refers to a compound to be tested by one or more screening method(s) of the invention, e.g., to determine if it is a putative modulator of an enzymatic activity such as a kinase activity. A test compound can be any chemical, such as an inorganic chemical, an organic molecule, a polypeptide, a carbohydrate, a polynucleotide, a polysaccharide, a lipid, a phospholipid, or a combination thereof. Typically, various predetermined concentrations (e.g., various dilutions) of test compounds are used for screening, such as 0.01 micromolar, 1 micromolar, or 10 micromolar. Experimental controls for a test compound can include measuring a signal for an assay performed in the absence of the test compound or comparing a signal obtained using a compound known to modulate a target activity with a signal obtained with the test compound.

## Binding Partners

The invention is based on monitoring and/or measuring a molecular interaction (e.g., complex formation or disruption) between two binding partners. A "binding partner" is a compound (e.g., a first binding partner) that has affinity for another compound (e.g., a second binding partner) (or vice versa) such that the two binding partners are capable of forming a complex when bound. Two binding partners can be members of a specific binding pair. For example, a first binding partner can be a monoclonal antibody and a second binding partner can be a composition having the epitope recognized by that monoclonal antibody.

Accordingly, in one aspect, the invention provides an article of manufacture comprising: packaging material; a first binding partner and a second binding partner. The first binding partner can comprise a luminescent metal complex, namely a Tb(III)-containing metal complex, while the second binding partner can comprise a fluorescent acceptor moiety, namely a GFP. Alternatively, the first binding partner can comprise a said fluorescent acceptor moiety, while the second binding partner can comprise a said luminescent metal complex.

Typically, the affinitiy (apparent $K_d$) of a first binding partner for a second binding partner is about 1 mM or less, e.g. about 10 $\mu$M or less, or about 1 $\mu$M or less, or about 0.1 $\mu$M or less, or 10 nM or less, or 1 nM or less, or 0.1 nM or less. As one of skill in the art will recognize, one can systematically adjust experimental parameters, e.g., concentrations of assay components, reaction times, temperatures, and buffers, depending on the $K_d$ of the first binding partner for the second binding partner, to obtain a desired combination of conditions and cost-effectiveness.

A second binding partner need not be an optimal binding partner form first binding partner. The term encompasses all binding partners whose binding interactions can be probed using the methods of the present invention. A second binding partner is sometimes referred to herein as a "tracer," and if it includes a luminescent metal complex or a fluorescent accept moiety, a "luminescent tracer."

A binding partner can be a polypeptide, a polynucleotide, a lipid, a phospholipid, a polysaccharide, or an organic molecule. Examples of specific polypeptide binding partners include an antibody, a protein, or an enzymatically or chemically-synthesized or modified polypeptide sequence (e.g. a polypeptide sequence derived from a protein, modified from a protein, or designed and synthesized *de novo*). A poypeptide binding partner may be linear or cyclic. An organic molecule binding partner can be a small organic molecule.

Typical examples of first and second binding partners that form complexes include an antibody and a composition having an epitope or epitope mimetic recognized by that antibody; a polypeptide and a ligand (e.g. receptor-ligand interactions); a polypeptide and another polypeptide (e.g., protein-protein interactions); a polypeptide and a polynucleotide (e.g. protein-DNA or protein-RNA interactions); a polynucleotide and another polynucleotide (e.g., DNA-DNA, DNA-RNA, or RNA-RNA interactions); a polypeptide and an organic molecule (e.g. protein-drug interactions); a polypeptide and a lipid (e.g. protein-phospholipid interactions); a polynucleotide and an organic molecule; and an organic molecule and another organic molecule.

A binding partner can comprise either a luminescent metal complex, namely a Tb(III)-containing metal complex, or a fluorescent acceptor moiety, namely a GFP. One binding partner comprises a said luminescent metal complex and the other comprises a said fluorescent acceptor moiety, e.g. a first binding partner comprises a said luminescent metal complex and a second binding partner comprises a said fluorescent acceptor moiety. Inclusion of a luminescent metal complex and fluorescent acceptor moiety on a binding partner pair allows an interaction of first and second binding partners to be monitored by one or more fluorescent techniques (e.g., FP, TR-RET, or multiplex modes). For example, when a first binding partner and second binding partner are bound to one another, the complex will typically exhibit a characteristic FP or TR-RET signal (or both). Disruption of the molecular interaction between the first binding partner and the second binding partner (e.g. by the addition of a competitor of the second binding partner) alters the FP or TR-RET signal (or both), allowing the monitoring of the molecular interaction in either FP or TR-RET modes (or both modes).

In one embodiment, an antibody can be labeled with a said luminescent metal chelate and a polypeptide binding partner for the antibody can be labeled with a said fluorescent acceptor moiety. When the antibody and polypeptide are bound to one another, the sample typically exhibits a high FP measurement and a fluorescence emission measurement characteristic of RET between the luminescent metal chelate and the acceptor moiety. Addition of a competitor at a suitable concentration and with a suitable $K_d$ for the antibody results in displacement of the second binding partner, with a concomitant reduction in the FP measurement of the sample and a change in the fluorescence emission measurement as a result of a loss of RET between the luminescent metal chelate on the antibody and the fluorescent acceptor moiety on the polypeptide.

Binding partners can be prepared and purified by a number of methods known to those of ordinary skill in the art. For example, antibodies, including monoclonal antibodies and antibody fragments, can be prepared by a number of methods known to those of skill in the art, or can be purchased from a variety of commercial vendors, including Serotec (Raleigh, NC), Abcam (Cambridge, MA), R&D Systems, Cambridge Antibody Technologies, and Covance Research Products (Denver, CO).

In general, an antigen for which an antibody is desired is prepared, e.g., recombinantly, by chemical synthesis, or by purification of a native protein, and then used to immunize animals. For example, polypeptides containing a particular amino acid sequence and/or post-translational modification (e.g., phosphorylation) can be prepared by solid-phase chemical synthesis in order to raise an antibody specific for the sequence and/or post-translational modification. Various host animals including, for example, rabbits, chickens, mice, guinea pigs, goats, and rats, can be immunized by injection of the antigen of interest. Depending on the host species, adjuvants can be used to increase the immunological response and include Freund's adjuvant (complete and/or incomplete), mineral gels such as aluminum hydroxide, surface-active substances such as lysolecithin, pluronic polyols, polyanions, peptides, oil emulsions, keyhole limpet hemocyanin, and dinitrophenol. Polyclonal antibodies are contained in the sera of the immunized animals. Monoclonal antibodies can be prepared using standard hybridoma technology. In particular, monoclonal antibodies can be obtained by any technique that provides for the production of antibody molecules by continuous cell lines in culture as described, for example, by Kohler et al. (1975) Nature 256:495-497, the human B-cell hybridoma technique of Kosbor et al. (1983) Immunology Today 4:72, and Cote et al. (1983) Proc. Natl. Acad. Sci. USA 80:2026-2030, and the EBV-hybridoma technique of Cole et al., Monoclonal Antibodies and Cancer Therapy, Alan R. Liss, Inc. pp. 77-96 (1983). Such antibodies can be of any immunoglobulin class including IgM, IgG, IgE, IgA, IgD, and any subclass thereof. The hybridoma producing the monoclonal antibodies of the invention can be cultivated *in vitro* or *in* vivo. Chimeric antibodies can be produced through standard techniques.

Antibody fragments that have specific binding affinity for an antigen can be generated by known techniques. Such antibody fragments include, but are not limited to, F(ab')$_2$ fragments that can be produced by pepsin digestion of an antibody molecule, and Fab fragments that can be generated by reducing the disulfide bridges of F(ab')$_2$ fragments. Alternatively, Fab expression libraries can be constructed. See, for example, Huse et al. (1989) Science 246:1275-1281. Single chain Fv antibody fragments are formed by linking the heavy and light chain fragments of the Fv region via an amino acid bridge (e.g., 15 to 18 amino acids), resulting in a single chain polypeptide. Single chain Fv antibody fragments can be produced through standard techniques, such as those disclosed in U.S. Patent No. 4,946,778.

Once produced, antibodies or fragments thereof can be tested for recognition of (and affinity for) a second binding partner by standard immunoassay methods including, for example, enzyme-linked immunosorbent assay (ELISA) or radioimmuno assay (RIA). See, Short Protocols in Molecular Biology, eds. Ausubel et al., Green Publishing Associates and John Wiley & Sons (1992). Suitable antibodies typically will have a $K_d$ for a second binding partner of about 1 mM or less, e.g., about 10 $\mu$M or less, or about 1 $\mu$M or less, or about 0.1 $\mu$M or less, or about 10 nM or less, or about 1 nM or less, or about 0.1 nM or less. For example, if a post-translationally modified protein is used to immunize an animal to produce an antibody specific for the particular post-translational modification, the second binding partner can be a polypeptide containing the same post-translational modification. In other embodiments, a second binding partner will have the same chemical structure as an antigen used to immunize.

Other polypeptides in addition to antibodies are useful as first or second binding partners and can also be prepared and analyzed using standard methods. By way of example and not limitation, polypeptides can be obtained by extraction from a natural source (e.g., from isolated cells, tissues or bodily fluids), by expression of a recombinant nucleic acid encoding the polypeptide, or by chemical synthesis. Polypeptides can be produced by, for example, standard recombinant technology, using expression vectors encoding the polypeptides. The resulting polypeptides then can be purified. Expression systems that can be used for small or large scale production of polypeptides include, without limitation, microorganisms such as bacteria (e.g., *E. coli* and *B. subtilis*) transformed with recombinant bacteriophage DNA, plasmid DNA, or cosmid DNA expression vectors; yeast (e.g., *S. cerevisiae*) transformed with recombinant yeast expression vectors; insect cell systems infected with recombinant virus expression vectors (e.g., baculovirus); plant cell systems infected with recombinant virus expression vectors (e.g., tobacco mosaic virus) or transformed with recombinant plasmid expression vectors (e.g., Ti plasmid); or mammalian cell systems (e.g., primary cells or immortalized cell lines such as COS cells, Chinese hamster ovary cells, HeLa cells, human embryonic kidney 293 cells, and 3T3 LI cells) harboring recombinant expression constructs containing promoters derived from the genome of mammalian cells (e.g., the metallothionein promoter) or from mammalian viruses (e.g., the adenovirus late promoter and the cytomegalovirus promoter).

Suitable methods for purifying the polypeptides can include, for example, affinity chromatography, immunoprecipitation, size exclusion chromatography, and ion exchange chromatography. See, for example, Flohe et al. (1970) Biochim. Biophys. Acta. 220:469-476, or Tilgmann et al. (1990) FEBS 264:95-99. The extent of purification can be measured by any appropriate method, including but not limited to: column chromatography, polyacrylamide gel electrophoresis, or high-performance liquid chromatography.

Polypeptides as first or second binding partners can also be prepared using solid phase synthesis methods, see, e.g., WO 03/01115 and 6,410,255. For ease of synthesis and cost considerations, it is preferred that polypeptides synthesized chemically have between 3 to 50 amino acids (e.g., 3 to 30, 3 to 20, 3 to 15, 5 to 30, 5 to 20, 5 to 15, 8 to 20, 8 to 15,10 to 10,10 to 15 or 10 to 12 amino acids in length). In the polypeptides useful in the invention, a great variety of amino acids can be used. Suitable amino acids include natural, non-natural, and modified (e.g., phosphorylated) amino acids. Amino acids with many different protecting groups appropriate for immediate use in the solid phase synthesis of peptides are commercially available.

Polynucleotides useful as binding partners can be produced by standard techniques, including, without limitation, common molecular cloning and chemical nucleic acid synthesis techniques. For example, polymerase chain reaction (PCR) techniques can be used. PCR refers to a procedure or technique in which target nucleic acids are enzymatically amplified. Sequence information from the ends of the region of interest or beyond typically is employed to design polynucleotide primers that are identical in sequence to opposite strands of the template to be amplified. PCR can be used to amplify specific sequences from DNA as well as RNA, including sequences from total genomic DNA or total cellular RNA. Primers are typically 14 to 40 nucleotides in length, but can range from 10 nucleotides to hundreds of nucleotides in length. General PCR techniques are described, for example in PCR Primer: A Laboratory Manual, ed, by Dieffenbach and Dveksler, Cold Spring Harbor Laboratory Press, 1995. When using RNA as a source of template, reverse transcriptase can be used to synthesize complementary DNA (cDNA) strands. Ligase chain reaction, strand displacement amplification, self-sustained sequence replication, or nucleic acid sequence-based amplification also can be used to obtain isolated nucleic acids. See, for example, Lewis Genetic Engineering News, 12(9):1 (1992); Guatelli et al., Proc. Natl. Acad. Sci. USA, 87:1874-1878 (1990); and Weiss, Science, 254:1292 (1991).

Polynucleotides useful in the invention also can be chemically synthesized, either as a single nucleic acid molecule (e.g., using automated DNA synthesis in the 3' to 5' direction using phosphoramidite technology) or as a series of smaller polynucleotides. For example, one or more pairs of long polynucleotides (e.g., >100 nucleotides) can be synthesized that contain the desired sequence, with each pair containing a short segment of complementarity (e.g., about 15 nucleotides) such that a duplex is formed when the polynucleotide pair is annealed. DNA polymerase is used to extend the polynucleotides, resulting in a single, double-stranded polynucleotide.

Polynucleotides useful in the invention also can be obtained by mutagenesis. For example, polynucleotides can be mutated using standard techniques including polynucleotide-directed mutagenesis and site-directed mutagenesis through PCR. See Short Protocols in Molecular Biology, Chapter 8, Green Publishing Associates and John Wiley & Sons, edited by Ausubel et al., 1992.

## Luminescent Metal Complex

A binding partner comprises a luminescent metal complex, namely a Tb(III)-containing metal complex. A luminescent metal complex can act as a donor fluorophore in a RET or TR-RET assay. A luminescent metal complex is useful in the present methods because its excited state lifetime is typically on the order of milliseconds or hundreds of microseconds rather than nanoseconds; a long excited state lifetime allows detection of a molecular interaction between binding partners to be monitored after the decay of background fluorescence and/or interference from light-scattering

Methods for covalently linking a luminescent metal complex to a variety of binding partners are known to those of skill in the art, see, e.g., WO 96/23526; WO 01/09188, WO 01/08712, and WO 03/011115; and U.S. Pat. Nos. 5,639,615; 5,656,433; 5,622,821; 5,571,897; 5,534,622; 5,220,012; 5,162,508; and 4,927,923.

A luminescent metal complex includes a metal liganding moiety, one or more lanthanide metal ions, and optionally linkers, spacers, and organic antenna moieties. The complexes useful in the invention contain Tb(III).

*Metal Liganding Moiety*

A metal liganding moiety coordinates one or more lanthanide metal ions to form a metal complex containing Tb(III). Typically, a metal liganding moiety includes one or more metal coordinating moieties X, where X is a heteroatom electron-donating group capable of coordinating a metal cation, such as O', OH, $NH_2$, $OPO_3^{2-}$, NHR, or OR where R is an aliphatic group.

A metal liganding moiety can be a chelating moiety or a cryptand moiety. If a lanthanide metal ion is coordinated to a chelating moiety, the complex is referred to as a "metal chelate." If a lanthanide metal ion is coordinated to a cryptand moiety, the complex is referred to as a "metal cryptand."

A metal chelate should be stable to exchange of the lanthanide ion. Metal chelates preferably have a formation constant ($K_f$) of greater than $10^{10}$M-1. A variety of useful chelating moieties are known to those of skill in the art. Typical examples of chelating moieties include: EDTA, DTPA, TTHA, DOTA, NTA, HDTA, DTPP, EDTP, HDTP, NTP, DOTP, DO3A, DOTAGA, and NOTA.

In some embodiments, a luminescent metal chelate can have the following structures:

$$-L_n-A-S_n-C_M,$$

or

$$-L_n-C_M-S_n-A,$$

wherein A represents an organic antenna moiety;
L represents a linker;
S represents a spacer;
n can be 0 or 1;
C represents a metal chelating moiety; and
M represents a Tb(III) metal ion coordinated to C.

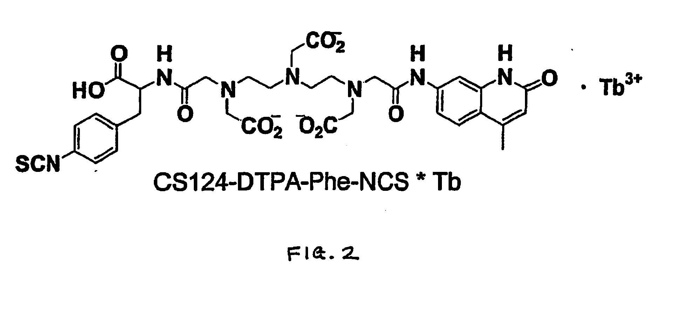

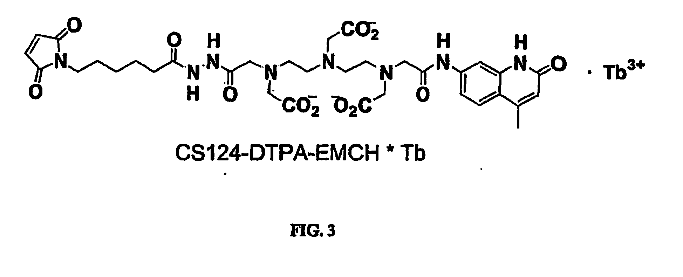

For illustrative examples of luminescent metal chelates, see FIGs. 2 and 3. FIG. 3 also demonstrates luminescent metal chelates useful for conjugating to amine moieties (top structure) or thiol moieties (bottom structure) on binding partners.

Cryptates are formed by the inclusion of a lanthanide cation into a tridimensional organic cavity, leading to highly stable complexes. A variety of useful cryptand moieties are known to those of skill in the art. Examples of cryptand moieties useful in the present methods include: trisbypyridine (TBP, e.g, TBP pentacarboxylate), and pyridine bipyridine (e.g., pyridine bipyridine tetracarboxylate).

Chelating and cryptand moieties can be synthesized by a variety of methods known to those of skill in the art or may be purchased commercially. See U.S. Pat. Nos. 5,639,615; 5,656,433; 5,622,821; 5,571,897; 5,534,622; 5,220,012; 5,162,508; and 4,927,923; and WO 96/23526 and WO 03/011115.

*Lanthanide Metal Ions*

Metal liganding moieties coordinate one or more lanthanide metal ions to form a metal complex containing Tb (III) Lanthanide metal ions are useful because their special electronic configuration shields the optically active electrons, resulting in characteristic line type emissions. As the electronic transitions of the metal ions are forbidden by quantum mechanics rules, the emission lifetimes of these ions are typically long (from $\mu$s to msec).

Useful lanthanide metal ions include Sm(III), Ru(III), Eu (III), Gd(III), Tb(III), and Dy(III). The metal complexes useful in the invention contain Tb(III). Methods for complexing a metal ion to a chelating or cryptand moiety are known to those of skill in the art, see, e.g., WO 96/23526 and WO 03/011115.

*Organic Antenna Moiety*

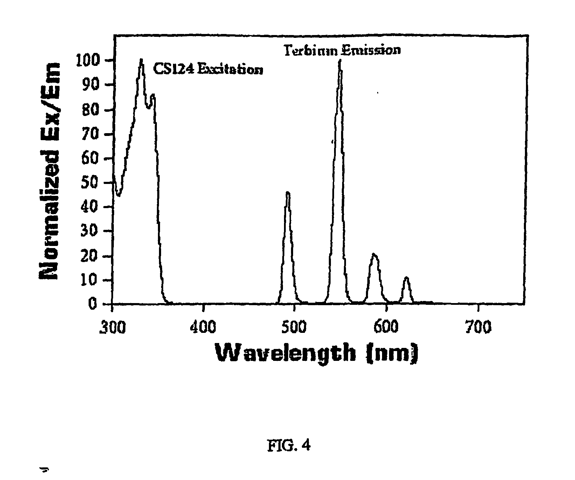

A luminescent metal complex can optionally include an organic antenna moiety. An organic antenna moiety typically has a conjugated electronic structure so that it can absorb light. The absorbed light is transferred by intramolecular non-radiative processes from the singlet to the triplet excited state of the antenna moiety, then from the triplet state to the emissive level of the lanthanide ion, which then emits characteristically long-lived luminescence. See FIG. 4. It should be noted that some metal liganding moieties can absorb light without the inclusion of an organic antenna moiety. For example, certain cryptand moieties that contain conjugated organic moieties, such as tribipyridine pentacarboxylate, do not require the inclusion of a discrete organic antenna moiety.

In some embodiments, an organic antenna moiety can be a polynuclear heterocyclic aromatic compound. The polynuclear heterocylic aromatic compound can have two or more fused ring structures. Examples of useful organic antenna moieties include rhodamine 560, fluorescein 575, fluorescein 590, 2-quinolone, 4-quinolone, 4-trifluoromethylcoumarin (TFC), 7-diethyl-amino-coumarin-3-carbohydrazide, 7-amino-4-methyl-2-coumarin (carbostyril 124, CS124), 7-amino-4-methyl-2-coumarin (coumarin 120), 7-amino-4-trifluomethyl-2-coumarin (coumarin 124), and aminomethyltrimethylpsoralen. See FIGs. 2 and 3.

Compounds useful as organic antenna moieties can be synthesized by methods known to those of skill in the art or purchased commercially. See U.S. Pat. Nos. 5,639,615; 5,656,433; 5,622,821; 5,571,897; 5,534,622; 5,220,012; 5,162,508; and 4,927,923.

### Linkers, Spacers

Linkers and Spacers can optionally be included in a luminescent metal complex. A Linker (L) functions to link a luminescent metal complex to a first or second binding partner. In some embodiments, a L can link an acetate, amine, amide, carboxylate, or methylene functionality on a metal liganding moiety to a first or second binding partner.

One of skill in the art can design Ls to react with a number of functionalities on binding partners, including, without limitation, amines, acetates, thiols, alcohols, ethers, esters, ketones, and carboxylates. In embodiments where the binding partner is a polypeptide, a L can cap the N-terminus, the C-terminous, or both N- and C- termini, as an amide moiety. Other exemplary L capping moieties include sulfonamides, ureas, thioureas and carbamates. Ls can also include linear, branched, or cyclic alkanes, alkenes, or alkynes, and phosphodiester moieties. The L may be substituted with one or more functional groups, including ketone, ester, amide, ether, carbonate, sulfonamide, or carbamate functionalities. Specific Ls contemplated also include NH--CO-NH-; -CO-$(CH_2)_n$-NH-, where n=1 1 to 10; -NH-Ph-; -NH-$(CH_2)_n$-, where n=1 to 10; -CO-NH-; - $(CH_2)_n$-NH-, where n=1 to 10; -CO-$(CH_2)_n$-NH-, where n=1 to 10; and -CS-NH-. Additional examples of Ls and synthetic methodologies for incorporating them into metal complexes, particularly metal complexes linked to polypeptides, are set forth in WO 01/09188, WO 01/08712, and WO 03/011115.

A Spacer (S) can connect an organic antenna moiety to a metal liganding moiety. In some embodiments, a S can link an acetate, amine, or methylene functionality on a metal liganding moiety to an organic antenna moiety One of skill in the art can design Ss to react with a number of functionalities on organic antenna moieties and on metal liganding moieties, including, without limitation, amines, acetates, thiols, alcohols, ethers, esters, ketones, and carboxylates. Ss can include linear, branched, or cyclic alkanes, alkenes, or alkynes, and phosphodiester moieties. The S may be substituted with one or more functional groups, including ketone, ester, amide, ether, carbonate, sulfonamide, or carbamate functionalities. Specific Ss contemplated also include NH--CO-NH-; -CO-$(CH_2)_n$-NH-, where n=1 to 10; -NH-Ph-; -NH-$(CH_2)_n$-, where n= 1 to 10; -CO-NH-; - $(CH_2)_n$-NH-, where n= 1 to 10; -CO-$(CH_2)_n$-NH-, where n=1 to 10; and -CS-NH-.

### Fluorescent Acceptor Moiety

A fluorescent acceptor moiety can act as an acceptor in RET or TR-RET-based assays and/or can be a fluorophore for which the polarization of fluorescence emission is measured in an FP-based assay.

In general, a fluorescent acceptor moiety should exhibit a good quantum yield and a large extinction coefficient; should be resistant to collisional quenching and bleaching; and should be easily conjugated to a variety of first and second binding partners by methods known to those having ordinary skill in the art.

GFP and GFP mutants are particularly useful in applications using Tb(III)-containing metal complexes. A variety of mutants of GFP from *Aequorea victoria* have been created that have distinct spectral properties, improved brightness, and enhanced expression and folding in mammalian cells compared to the native GFP (e.g., see Table 7 of U.S. 6,410,255 and also Green Fluorescent Proteins, Chapter 12, pages 19 to 47, edited by Sullivan and Kay, Academic Press; U.S. patent Nos: 5,625,048 to Tsien et al., issued April 29, 1997; 5,777,079 to Tsien et al., issued July 7, 1998; and U.S. Patent No. 5,804,387 to Cormack et al., issued September 8, 1998). Table 7 of U.S. 6,410,255 lists the following mutations:

| Mutations | Common Name |
|---|---|
| S65 type | |
| S65T, S72A, N149K, M153T, I167T | Emerald |
| F64L, S65T, V163A | |
| F64L, S65T (EGFP) | EGFP |
| S65T | |

(continued)

| Y66H type | |
|---|---|
| F64L, Y66H, Y145F, V163A | P4-3E |
| F64L, Y66H, Y145F | |
| Y66H, Y145F | P4-3 |
| Y66H | BFP |
| Y66W type | |
| S65A, Y66W, S72A, N1461, M153T, V163A | W1C |
| F64L, S65T, Y66W, N1461, M153T, V163A | W1B |
| Y66W, N146I, M153T, V163A | hW7 |
| Y66W | |
| T203Y type | |
| S65G, S72A, K79R, T203Y | Topaz |
| S65G, V68L, S72A, T203Y | 10C |
| S65G, V68L, Q69K, S72A, T203Y | h10C+ |
| S65G, S72A, T203H | |
| S65G, S72A, T203F | |
| T203I type | |
| T203I, S72A, Y145F | Sapphire |
| T203I, T202F | H9 |

A fluorescent acceptor moiety for use in multiplex assays should exhibit characteristics useful for both RET/TR-RET applications and FP applications. For example, for FP assays, a fluorophore preferably exhibits a fluorescent excited state lifetime of at least 1 nanosecond, or at least 2 nanoseconds. For TR-RET applications, a region of the fluorophore's absorbance spectrum should overlap with a region of a luminescent metal chelate's emission spectrum, while a region of the fluorophore's emission spectrum should not overlap substantially with a region of the luminescent metal chelate's emission spectrum.

Methods for incorporating fluorophores into a variety of binding partners are known to those of skill in the art; see, e.g , US 6,410,255.

### Methods and Assays

Methods of the present invention are based on the finding that luminescent or fluorescent assays based on FP or RET (including TR-RET) can be easily converted into multiplex assays, allowing detection in multiple luminescent modes. For example, assays based on measuring a change in FP of one binding partner can be easily converted to TR-RET assays by the incorporation of an appropriate luminescent Tb(III)-containing metal complex on one binding partner and a GFP acceptor moiety on the other binding partner. In some embodiments, a binding partner that includes a fluorescent acceptor moiety useful for FP measurements need not be modified, provided that a suitable luminescent metal complex is chosen for inclusion on the other binding partner, as described above. Any of the methods described herein can be homogeneous or heterogeneous.

### Fluorescence Polarization

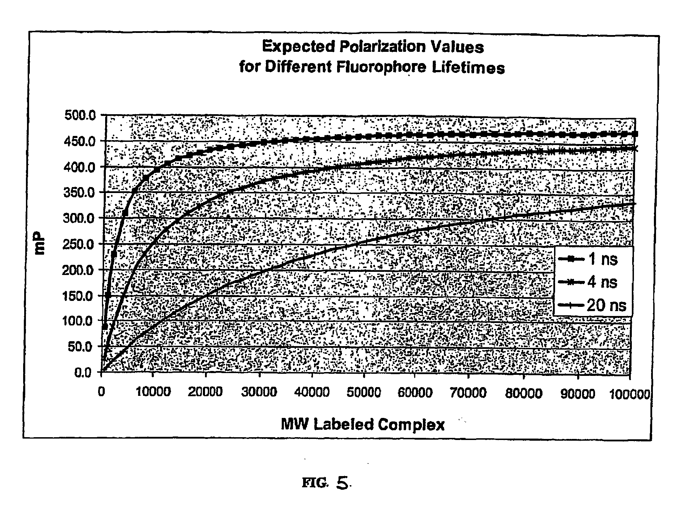

Methods of the present invention take advantage of a change in fluorescence polarization upon first and second binding partner complex formation or disruption. Polarization measurements are based on the relative rotational movement of a fluorophore compared to the excited state life-time of that fluorophore. For globular molecules in dilute solution, the relationship between polarization (p) and the degree of rotational movement can be readily derived (see Weber, Polarization of the fluorescence of solutions, in Fluorescence and Phosphorescence Analysis, Don Hercules (ed.), Interscience Publishers, New York, Chapter 8, pages 217-240 (1966)). Rotational movement can be related to the rotational diffusion constant of the molecule, and hence to the molecular volume. In practice there is often a close correlation between molecular size and relative polarization of emitted light from a fluorophore, although effects due to conformational freedom of a molecule, even when bound, are hard to predict. See FIG. 5 for a graph of expected polarization values for different fluorophore lifetimes and MWs of complexes of a first and second binding partner.

A change in the fluorescence polarization of a sample can occur when a complex of a first and second binding partner (one of which comprises a fluorescent acceptor moiety) is either formed or disrupted. Complex formation or disruption can result in from about a 10% to about a 10,000% increase or decrease in the fluorescence polarization measurement of a sample. Preferably, a change in the fluorescence polarization measurement is from about 30 mP to about 450 mP.

Polarization-based assays are relatively easy to set up and can be obtained over a wide concentration, temperature, and ionic strength range. See, e.g., U.S. 6,511,815 and 5,445,935.

### RET and TR-RET

Methods of the present invention also take advantage of resonance energy transfer between a luminescent metal chelate, namely a Tb(III)-containing metal complex, and a fluorescent acceptor moiety (RET), namely a GFP. A donor luminescent metal chelate is excited by light of appropriate wavelength and intensity (e.g., within the donor antenna moiety's excitation spectrum) and under conditions in which direct excitation of the acceptor fluorophore is minimized. The donor luminescent chelate then transfers the absorbed energy by non-radiative means to the acceptor fluorescent moiety, which subsequently re-emits some of the absorbed energy as fluorescence emission at one or more characteristic wavelengths. In TR-RET applications, the re-emitted radiation is not measured until after a suitable delay time, e.g., 26, 50, 75, 100, 150, 200, or 300 microseconds, to allow decay of background fluorescence, light scattering, or other luminescence, such as that caused by the plastics used in microtiter plates.

In some RET applications, a first binding partner can comprise either a luminescent metal complex or a fluorescent acceptor moiety, while the second binding partner comprises the other. For example, but outside the scope of the invention, an antibody first binding partner can be labeled with a Tb(III)-chelate-organic antenna moiety (luminescent metal chelate), while a polypeptide for which the antibody is specific can be labeled with a fluorescein (fluorescent acceptor moiety). In this case, disruption of the complex formed by the antibody and polypeptide (e.g., by a compound that affects binding between the two) results in an alteration in energy transfer between the luminescent metal chelate on the antibody and the fluorescent acceptor moiety on the polypeptide that may be used to monitor and measure the binding between the first and second binding partners. A compound that affects binding of a second binding partner (or tracer) to a first binding partner can be, for example, a test compound, an enzyme product (e.g., for which the first binding partner has specificity), or an enzyme substrate (e.g., for which the first binding partner has specificity).

In other RET embodiments, a compound that affects binding of a second binding partner (or tracer) to a first binding partner can comprise either a luminescent metal chelate or fluorescent acceptor moiety while the first binding partner comprises the other. In these embodiments, disruption of the complex formed between the first binding partner and the second binding partner by the labeled compound that affects binding can result in an increase in RET.

RET can be manifested as a reduction in the intensity of the luminescent signal from the donor luminescent metal complex and/or an increase in emission of fluorescence from the acceptor fluorescent moiety. For example, when a complex between an antibody having a donor luminescent metal complex and a polypeptide having an acceptor fluorescent moiety is disrupted, e.g., by a competitor for the polypeptide, such as an unlabeled polypeptide, the donor luminescent metal complex and the acceptor fluorescent moiety physically separate, and RET is diminished or eliminated. Under these circumstances, luminescence emission from the donor luminescent metal complex increases and fluorescence emission from the acceptor fluorescent moiety decreases. Accordingly, a ratio of emission amplitudes at wavelengths characteristic (e.g., the emission maximum) of the donor luminescent metal complex relative to the acceptor fluorescent moiety should increase as compared to the same ratio under RET conditions (e.g., when emission of the donor luminescent metal complex is quenched by the acceptor).

The efficiency of RET is dependent on the separation distance and the orientation of the donor luminescent metal complex and acceptor fluorescent moiety, the luminescent quantum yield of the donor metal ion, the spectral overlap with the acceptor fluorescent moiety, and the extinction coefficient of the acceptor fluorophore at the wavelengths that overlap with the donor's emission spectra. Forster derived the relationship:

$$E = (F^\circ - F)/F^\circ = Ro^6/(R^6 + Ro^6)$$

where E is the efficiency of RET, F and F° are the fluorescence intensities of the donor in the presence and absence of the acceptor, respectively, and R is the distance between the donor and the acceptor. Ro, the distance at which the energy transfer efficiency is 50% of maximum is given (in Å) by:

$$Ro = 9.79 \times 10^3 (K^2 QJn^{-4})^{1/6}$$

where $K^2$ is an orientation factor having an average value close to 0.67 for freely mobile donors and acceptors, Q is the quantum yield of the unquenched fluorescent donor, n is the refractive index of the intervening medium, and J is the overlap integral, which expresses in quantitative terms the degree of spectral overlap. The characteristic distance Ro at which RET is 50% efficient depends on the quantum yield of the donor, the extinction coefficient of the acceptor, the overlap between the donor's emission spectrum and the acceptor's excitation spectrum, and the orientation factor between the two fluorophores.

Changes in the degree of RET can be determined as a function of a change in a ratio of the amount of luminescence from the donor and acceptor moieties, a process referred to as "ratioing." By calculating a ratio, the assay is less sensitive to, for example, well-to-well fluctuations in substrate concentration, photobleaching and excitation intensity, thus making the assay more robust. This is of particular importance in automated screening applications where the quality of the data produced is important for its subsequent analysis and interpretation. See, e.g., U.S. 6, 410, 255; 4,822,733; 5,527,684; and 6,352,672.

For example, in some embodiments of the method, a ratiometric analysis is performed, wherein a ratio of luminescence emission at two different wavelengths is compared between a test sample and a control sample. In a typical TR-RET-based assay, the two wavelengths can correspond to an emission maximum for a luminescent metal complex and a fluorescent acceptor moiety. In some embodiments, an emissions ratio of the control sample will be about 1.5, 2, 3, 4, 5, 7, 10, 15, 20, 25, 30, 40, 50, or 100 times larger or smaller than the emissions ratio of a test sample.

***Methods for Measuring Effects of Test Compounds on Binding Between Binding Partners***

Methods of the present invention can be used to measure the effect of a test compound on binding between a first binding partner and a second binding partner. For example, the present methods may be used to identify competitive binders to first or second binding partners, or to identify compounds that physically (e.g., allosterically) or chemically affect a first or second binding partner so as to consequently affect binding of its partner. Accordingly, assays to identify effects of test compounds on such binding partner interactions as protein-protein interactions, protein-ligand interactions, protein-DNA interactions, and polynucleotide hybridizations may be designed using the present methods.

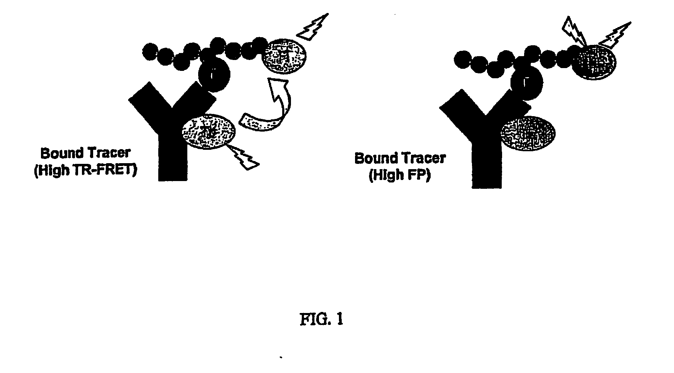

In one method, a first binding partner, a second binding partner, and a test compound are contacted to form a test sample. In some embodiments, one of the binding partners comprises a luminescent metal complex, namely a Tb (III)-containing complexes while the other comprises a fluorescent acceptor moiety, namely a GFP See FIG. 1. As described previously, the first and second binding partner are capable of binding to one another to form a complex. The test sample is exposed to polarized light at an appropriate wavelength (e.g., at a wavelength in an absorbance band of the fluorescent acceptor moiety) and the polarization of fluorescent emission from the test sample is measured. In some embodiments, the test sample is also exposed to light (e.g., at a wavelength in an absorbance band of the luminescent metal complex), typically in the wavelength range of 250 nm to 750 nm, and the fluorescence emission from the test sample is measured. Fluorescence emission may be measured after a suitable time delay, as indicated above, to result in a time-resolved fluorescence emission measurement.

In embodiments where a test sample is exposed to polarized light and also light (e.g., having a wavelength in the range from 250 nm to 750 nm), the exposure to polarized light and measurement of polarization may be performed before, after, or simultaneously with the exposure to light and measurement of fluorescence emission from the test sample. For example, the exposure to polarized light and measurement of polarization may be performed up to 5 sec., 10 sec., 20 sec., 30 sec., 1 min., 2 min., 5 min., 10 min., 1 hr., 5 hrs., or 24 hrs. before or after the exposure to light and measurement of fluorescence emission from the test sample.

In other embodiments, as explained above, a test compound can comprise either a said luminescent metal complex or a said fluorescent acceptor moiety and a first binding partner can comprise the other. For example, a first binding partner receptor can be labeled with a luminescent metal chelate while a test ligand for the first binding partner receptor can be labeled with a fluorescent acceptor moiety Disruption of a complex formed between the first binding partner receptor and an unlabeled second binding partner (e.g., a ligand for the receptor) by the labeled test ligand can lead to an increase in RET and/or FP.

A test compound is identified as affecting binding between first and second binding partners when the fluorescence polarization measurement or the fluorescence emission measurement of the test sample, or both, is different from the fluorescence polarization measurement or the fluorescence emission measurement of a control sample lacking the test compound. Generally, there should be a statistically significant difference in one or both measurements as compared to the control sample. As one of skill in the art will recognize, whether or not a difference is statistically significant will depend on the type of measurement and the experimental conditions. It is understood that when comparing measurements, a statistically significant difference indicates that the test compound may warrant further study. Typically, a difference is considered statistically significant at $p < 0.05$ with an appropriate parametric or non-parametric statistic, e.g., Chi-square test, Student's t-test, Mann-Whitney test, or F-test. In some embodiments, a difference is statistically significant at $p < 0.01$, $p < 0.005$, or $p < 0.001$.

### *Methods for Identifying Modulators of Enzymatic Activity*

Methods of the invention are used to identify a modulator of enzymatic activity. A first binding partner is selected based on specificity for either a substrate or a product of an enzymatic activity. For example, an antibody with specificity for a phosphorylated tyrosine as compared to an unmodified tyrosine can be a first binding partner with specificity for a product of tyrosine kinase activity. A tracer is then selected based partially on the specificity of the first binding partner for the substrate or product of the enzymatic activity. For example, a tracer can include the purported epitope recognized by an antibody first binding partner, or a recognition site or chemical structure recognized by a polypeptide first binding partner. In other embodiments, a tracer can have the same chemical structure as an antigen used to immunize an animal to generate a first binding partner antibody. Typically, the first binding partner will bind to a tracer with a similar $K_d$ as to the enzymatic product or substrate for which it has specificity, e.g., about 0.001 to 1000 times, or 0.01 to 100 times, or 0.1 to 10 times the $K_d$ of the first binding partner for the product or substrate.

A tracer may be labeled (e.g., include a luminescent Tb(III)-containing metal complex or a fluorescent GFP acceptor moiety; referred to herein as a "luminescent tracer") or the tracer may be unlabeled. For example, if the first binding partner is an antibody with specificity for a phosphorylated tyrosine, a product of tyrosine kinase activity, a luminescent tracer can be selected that includes the epitope (or an epitope mimetic) recognized by the antibody (in this case, a phosphorylated tyrosine) so that the antibody binds the luminescent tracer. The inclusion of a fluorescent acceptor moiety or luminescent metal complex on the tracer should not substantially affect the $K_d$ of the first binding partner for the tracer.

Because the assay is based on the selection of a first binding partner having specificity for a product or substrate of an enzymatic activity, a wide variety of enzymatic activities may be probed, including, without limitation, kinase activity, phosphatase activity, glucuronidase activity, prenylation, glycosylation, methylation, demethylation, acylation, acetylation, ubiquitination, sulfation, proteolysis, nuclease activity, nucleic acid polymerase activity, nucleic acid reverse transcriptase activity, nucleotidyl transferase activity, polynucleotide transcription activity, and polynucleotide translation activity.

In the method, an enzyme is contacted with a substrate for the enzyme under conditions effective for an enzymatic activity of the enzyme to form a product from the substrate. As one of skill in the art will recognize, conditions effective for enzymatic activity will vary with the enzyme, enzymatic activity, and substrate chosen. For kinase reactions, ATP is generally included. Incubation conditions for a contacting step can vary, e.g., in enzyme concentration, substrate concentration, temperature, and length of time. Incubation temperature conditions typically can be from about 15 to about 40 °C; in some embodiments, the temperature may be about room temperature, e.g., about 20-25 °C.

A contacting step is carried out in the presence of a potential modulator of the enzymatic activity. In some embodiments, the enzyme, substrate, and potential modulator mixture is then contacted with a first binding partner and luminescent tracer, as described above, to form a test sample. As indicated previously, in these embodiments, either the first binding partner or the luminescent tracer includes a said luminescent metal complex, while the other includes a said fluorescent acceptor moiety.

In other embodiments, the enzyme, substrate, and potential modulator mixture is contacted with a first binding partner and a tracer to form a test sample. In these embodiments, either the first binding partner or the substrate includes a luminescent Tb(III)-containing metal complex, while the other includes a fluorescent GFP acceptor moiety. In such cases, enzymatic activity can result in the conversion of the labeled substrate to a labeled product. The inclusion of a said fluorescent acceptor moiety or luminescent metal complex on the substrate should not substantially affect the ability of the enzyme to form a product from the labeled substrate. In addition, the inclusion of a said fluorescent acceptor moiety or luminescent metal complex on the substrate (or product) should not substantially affect the $K_d$ of the first binding partner for the substrate (or product) for which it has specificity.

The test sample is exposed to polarized light at an appropriate wavelength (e.g., at a wavelength in an absorbance band of the fluorescent acceptor moiety) and the polarization of fluorescent emission from the test sample is measured. In some embodiments, the test sample is also exposed to light (e.g., at a wavelength in an absorbance band of the luminescent metal complex), typically in the wavelength range of 250 nm to 750 nm, and the fluorescence emission from the test sample is measured. Fluorescence emission may be measured after a suitable time delay, as indicated above, to result in a time-resolved fluorescence emission measurement. As indicated previously, the exposure to polarized light and measurement of polarization may be performed before, after, or simultaneously with the exposure to light and measurement of fluorescence emission from the test sample.

As described above, in some embodiments a tracer may be unlabeled, e.g., in embodiments where a first binding partner is labeled with a said luminescent metal complex and a substrate is labeled with a said fluorescent acceptor moiety. Disruption of a complex formed between an unlabeled tracer and a labeled first binding partner by an appropriately labeled compound (e.g., labeled substrate, labeled product, labeled test compound) that affects binding between the unlabeled tracer and first binding partner can lead to an increase or decrease in RET, FP, or both.

A potential modulator is identified as a modulator of enzymatic activity when the fluorescence polarization measurement or the fluorescence emission measurement of the test sample, or both, is different from the fluorescence polarization measurement or the fluorescence emission measurement of a control sample lacking the potential modulator. As indicated above, there should be a statistically significant difference as compared to the control sample. As one of skill in the art will recognize, whether or not a difference is statistically significant will depend on the type of measurement and the experimental conditions. It is understood that when comparing measurements, a statistically significant difference indicates that that potential modulator may warrant further study. Typically, a difference is considered statistically significant at $p < 0.05$ with an appropriate parametric or non-parametric statistic, e.g., Chi-square test, Student's t-test, Mann-Whitney test, or F-test. In some embodiments, a difference is statistically significant at $p < 0.01$, $p < 0.005$, or $p < 0.001$.

Any of the methods of the present invention can be modified to be performed in a high-throughput or ultra-high-throughput manner. For example, a method to identify a modulator of activity of an enzyme may be modified to contact a plurality of substrates, independently, with a particular enzyme and potential modulator, to form a plurality of enzyme mixtures. Each enzyme mixture is then contacted with an appropriate first binding partner and luminescent tracer to form a test sample, with the excitation (e.g., exposure to plane polarized light and exposure to light) and measurement (of emission of polarized and fluorescent light) steps as described previously. As one of skill in the art will appreciate, such high-throughput methods are particularly amenable to multi-well plate or 2-D array panel formats. Devices for incubating and monitoring multi-well plates are known in the art.

The dynamic range, quality, and robustness of the methods of the rpesent invention can be evaluated statistically. For example, the Z'-Factor is a statistic designed to reflect both assay signal dynamic range and the variation associated with signal measurements. Signal-to-noise (S/N) or signal-to-background (S/B) ratios alone are unsatisfactory in this regard because they do not taken into account the variability in sample and background measurements and signal dynamic range. The Z'-Factor takes into account these factors, and because it is dimensionless, it can be used to compare similar assays. Typically, assays of the present invention yield Z'-factors of greater than or equal to 0.5. Methods for determining Z'-factor are known to those of skill in the art. A Z'-factor may be determined by evaluating the dynamic range of a method.

### *Articles of Manufacture and Apparatuses*

The invention also provides articles of manufacture, such as kits. The disclosure also provides apparatuses useful for performing the described inventions. Typically, a kit includes packaging materials, such as a container, and compositions useful as first and second binding partners. In some embodiments, a kit can include one or more of the following: a multi-well plate, one or more enzymes, buffers, and directions for use of the kit.

An apparatus will generally include a sample chamber, means for generating plane polarized light to illuminate the sample chamber; and means for illuminating the sample chamber with light having a wavelength from 250 nm to 750 nm. In addition, an apparatus will include means for detecting polarized light emitted from the sample chamber and means for detecting light (e.g., fluorescence) emitted from the sample chamber. Both illumination means may illuminate the sample chamber simultaneously. Alternatively, both detections means detect the polarized light and the light emitted (e.g., fluorescence) simultaneously.

### EXAMPLES

Example 1 describes preparation of a Tb(III)-labeled antibody useful in the invention. The remaining examples are not part of the claimed invention but, nonetheless, may assist in understanding the invention.

Example 1 - Labeling of Antibody with a Luminescent Metal Chelate

1mg purified PY72 (anti-phosphotyrosine) IgG antibody, an antibody that preferentially binds amino acid sequences containing phosphorylated tyrosines (e.g., sequences phosphorylated by protein tyrosine kinases (PTKs)) and was dialyzed for 1.5 hours in a 100 mM sodium bicarbonate buffer, pH 9.5, using a 12-14,000 MWCO dialysis membrane. [PY72 hybridoma cells were obtained from the Salk Institute; the immunogen was phosphotyrosine conjugated to KLH. Ascites were produced by Harlan Bioproducts for Science, Indianapolis IN. Ascites were purified with a protein G column (Pierce). Purified antibody is also available from Covance, Berkeley CA (Part # MMS414P).] The antibody was then removed from the dialysis membrane and concentrated to 48.8 uM (7.3 mg/mL) using a Centricon YM50 (Millipore) concentrator. 100 uL of this antibody solution was diluted to 5 mg/ml (33.4 uM) into the labeling reaction which consisted of 10 mM phenyl phosphate, and 660 $\mu$M carbostyril 124-diethylenetriaminepentaaceticacid-phenylalanine isothiocyanate *Tb(III)) (CS124-DTPA-Phe-NCS*Tb, see FIG. 3) (final concentrations) in 100 mM sodium bicarbonate buffer, pH 9.5. The reaction was incubated at room temperature for 4 hours with light vortexing every 30 minutes, and then dialyzed twice for 1.5 hours each against tris-buffered saline (TBS) to remove unreacted and/or hydrolyzed chelate. The amount of chelate bound to the antibody was quantitated by the absorbance of the CS 124 moiety at 343 nm ($E_{340}$ = 11,440 $M^{-1}cm^{-1}$), and the amount of antibody quantitated by its absorbance at 280 nm ($E_{280}$ = 210,000 $M^{-1}cm^{-1}$), correcting for the absorbance of the CS124 at 280 nM (1.1 times its absorbance at 343 nM). From these measurements it was determined that the reaction produced an antibody labeled with an average of 5.8 chelates per antibody.

A monoclonal antibody with specificity for phosphorylated serines (anti-pSer; phosphorylated serines are products of Serine/Threonine kinase activity) was also prepared and labeled with a luminescent metal chelate, as described above.

Example 2 - Binding Curve Experiment between Protein Tyrosine Kinase Product Tracer (PTK Tracer) and Anti-PTK Product (PY72) Antibody

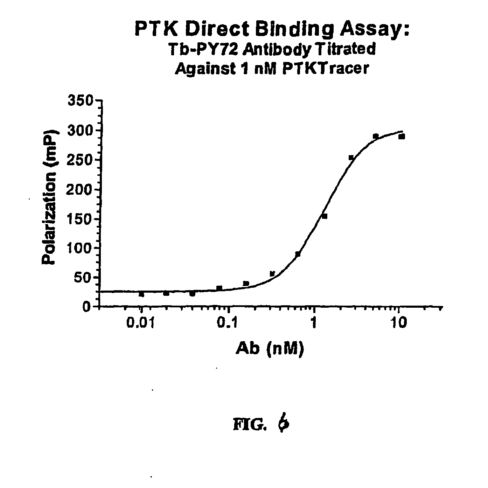

A direct binding curve (showing luminescent metal chelate -labeled PY72 antibody binding to fluorescent acceptor labeled tracer) was generated by incubating serial dilutions of the labeled antibody (10 nM to 9.8 pM in two fold dilutions) with 1 nM fluorescent acceptor-labeled tracer (PTK labeled tracer; sequence F-ADE(pY)LIPQQS, where F is fluorescein and pY is a phosphorylated tyrosine, SEQ ID NO:1; note that the tracer is a phosphorylated tyrosine derivative of a protein tyrosine kinase (PTK) substrate) in FP dilution buffer (PanVera, Madison WI part #P2839). After a 30 minute incubation, the fluorescence polarization of each composition in the plate was read on a Tecan Ultra plate reader using a 485 nm excitation filter (20 nm bandpass) and 535 nm emission filters (25 nm bandpass). Data was collected using 10 flashes per well and a 40 $\mu$s integration time. The antibody was seen to bind to the tracer with an EC50 of slightly more than 1 nM. See FIG. 6.

A similar binding curve was performed with a luminescent metal chelated-labeled anti-pSer antibody and a fluorescent acceptor-labeled tracer (STK labeled tracer, sequence F-GRPRTS(pS)FAEG, where F is a fluorescein and pS is a phosphorylated serine, SEQ ID NO:2; note that the tracer is a phosphorylated serine derivative of a S/T kinase (STK) substrate).

Example 3 - Competition Curve between Labeled Kinase Product Tracer and Unlabeled Kinase Product

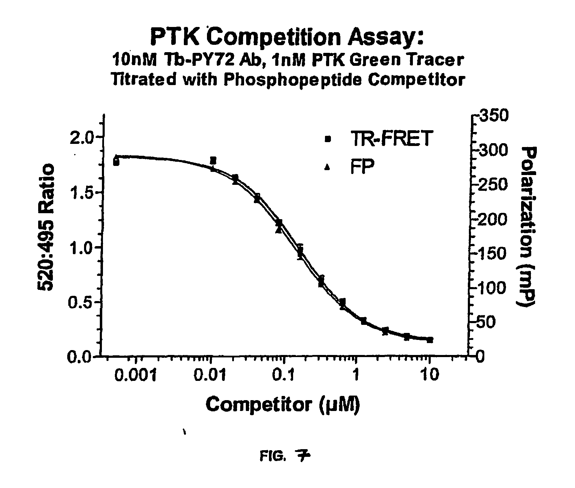

A competition curve to show that the disruption of the antibody-tracer interaction could be monitored by both fluorescence polarization and time-resolved RET from the same sample was performed by incubating serial dilutions (10 $\mu$M to 19.5 nM in two-fold dilutions) of an unlabeled phosphotyrosine-containing peptide competitor (ADE(pY) LIPQQS, where pY is a phosphorylated tyrosine, SEQ ID NO:3) in the presence of 10 nM Tb-chelate labeled PY72 antibody and 1 nM labeled PTK labeled tracer, as described above. After a 30 minute incubation, the plate was read on a Tecan Ultra plate reader. Fluorescence polarization was measured using a 485 nm excitation filter (20 nm bandpass) and 535 nm emission filters (25 nm bandpass). Time-resolved RET was measured using a 340 nm excitation filter (35 nm bandpass) and 495 nm (10 nm bandpass) and 520 nm (25 nm bandpass) filters using a 200 $\mu$s integration window after a 100 $\mu$s post-flash delay with 10 flashes per well. The time-resolved RET value (ratio) was calculated by dividing the 520 nm signal by the 495 nm signal. The shapes of the curves generated by TR-RET or FP were seen to nearly overlap, indicating that the presence of a phosphopeptide (such as that generated by a kinase reaction) could be detected and quantitated using FP or TR-RET, or both. See FIG. 7.

Example 4 - Screening of Test Compounds as Modulators of Kinase Activity using Multimode FP and TR-RET Measurements

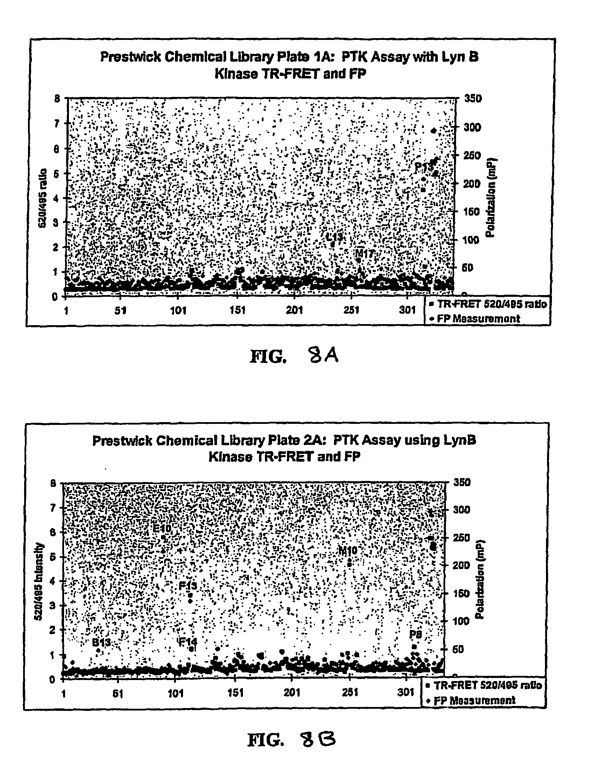

A chemical library screen to identify inhibitors of Lyn B Kinase, a member of the SRC family of protein tyrosine kinase (PTK) enzymes, was performed. The kinase reaction was performed in the presence of 10 µM of a Prestwick library compound (test compound; Prestwick Library available from Prestwick Chemical, Inc., Washington DC) in 20 mM HEPES pH 7.5, 5 mM MgCl$_2$, 150 nM poly(Gly:Tyr, 4:1) protein tyrosine kinase substrate, and 10 µM ATP using 1 ng of Lyn B kinase per reaction. The kinase reaction was allowed to proceed for 1 hour at room temperature and then stopped by adding 100 mM EDTA to a final concentration of 5 mM in a total volume of 40 µl. To detect the presence of phosphopeptide product, 10 µl of a solution (containing 20 nM Tb-chelate labeled PY72 antibody and 10 nM PTK labeled tracer was added to each well and incubated for an additional 30 min. The plate was then read on a Tecan Ultra plate reader in both fluorescence polarization and time-resolved RET measurement modes. Fluorescence polarization was measured using a 485 nm excitation filter (20 nm bandpass) and 535 nm emission filters (25 nm bandpass). Time-resolved RET was measured using a 340 nm excitation filter (35 nm bandpass) and 495 nm (10 nm bandpass) and 520 nm (25 nm bandpass) filters using a 200 µs integration window after a 100 µs post-flash delay with 10 flashes per well. The time-resolved RET value (ratio) was calculated by dividing the 520 nm signal by the 495 nm signal Kinase inhibitors were identified by wells that showed high polarization or 520:495 TR-RET ratios. The results of the screen of approximately 750 compounds are shown in FIG. 8.

Example 5 - Conversion of FP Assay to Multiplex FP/TR-RET Assay

Because terbium-chelates are able to serve as donors to fluorophores such as fluorescein or rhodamine (and derivatives thereof) in TR-RET assays, and because fluorescein and rhodamine have excellent properties for use in FP assays, it is a simple matter to modify an FP assay such that it can be read in a dual-mode FP/TR-RET manner by labeling, for example, a binding partner such as a receptor protein or an antibody with a fluorescent terbium chelate. The use of multiplex modes (e.g., both FP and TR-RET) allows verification of data and elimination of false positive or false negative results. In addition, assays that are problematic in either the FP mode or TR-RBT mode may be converted to robust assays using the other mode.

An FP assay to detect phosphorylation of Ser133 on the cyclic-AMP response element binding protein (CREB) by CREB kinase (a serine kinase) was designed. The assay required the identification of a fluorescein-labeled kinase product tracer containing a phosphorylated serine. In addition, the assay required an and-CREB pSer133 antibody (available from Cell Signaling Technologies, Beverly, MA) capable of binding the tracer. Four candidate tracer peptides were prepared, as shown below, and tested for binding to the anti-pSer133 antibody. The tracers differed in their length and in the position of the fluorophore on the peptide.

Tracer 1: Fluorescein-LRREILSIRRP(pS)YRK (SEQ ID NO:4);
Tracer 2: Fluorescein-REILSRRP(pS)YRK (SEQ ID NO:5)
Tracer 3: Fluorescein-ILSRRP(pS)YRK (SEQ ID NO:6); and
Tracer 4: LRREILSRRP(pS)YRK-Fluorescein (SEQ ID NO:7).

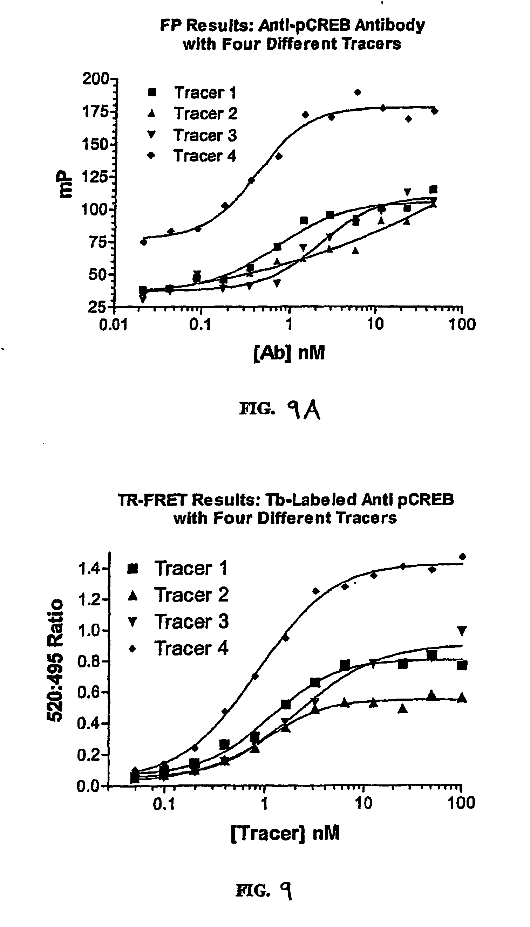

When tested in direct binding to the antibody in FP mode, two tracers were seen to bind with sub-nM Kd affinities, but neither showed a change in polarization greater than 100 mP between the free and bound state. See FIG. 9A. The robustness of an FP assay is in part a function of the magnitude of this difference in polarization. As changes in polarization of greater than 30 mP, or greater than 50 mP, or greater than 100 mP, are generally preferred, an attempt was made to convert the assay to a TR-RET assay.

The anti-pSer133 antibody was labeled with CS124-DTPA-Phe-NCS*Tb (see Example 1 above) to yield an antibody with an average of 6.2 chelate molecules per antibody. When the four candidate tracer peptides were titrated separately against this labeled antibody, SEQ ID NO:7 was seen to bind with sub-nM affinity and a 32-fold change in TR-RET value between free and bound forms. See FIG. 9B.

Example 6 - PKA Enzyme Titration Demonstrating Z'-Factor of TR-RET Assay

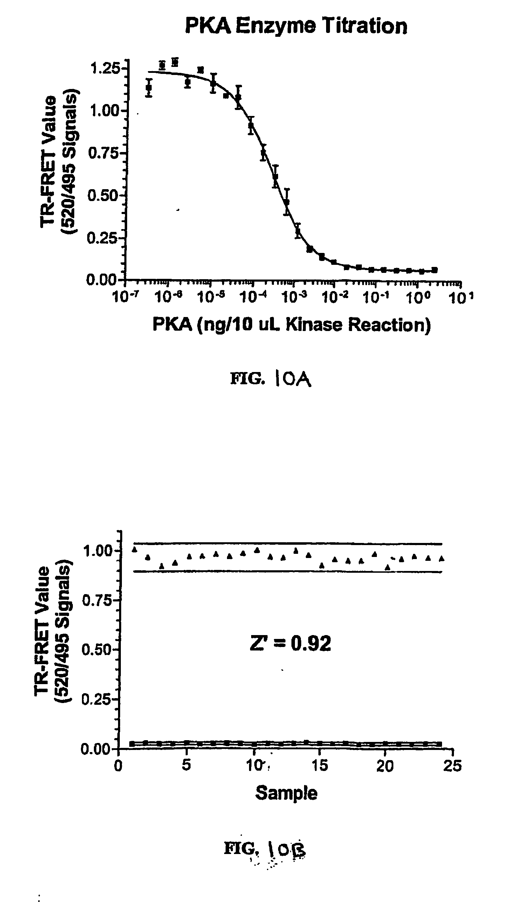

PKA (a serine kinase) was serially diluted across 24 wells of a 384 well plate and reacted with 1 µM peptide PKA substrate (LRREILSRRPSYRK, SEQ ID NO:8) in 50 mM Tris (pH 7.5) containing 10 mM MgCl$_2$, 50 µM NaVO$_4$, and 5 µM ATP. The final reaction volume was 10 (µL per well. The reactions were allowed to proceed for 90 minutes at room temperature, after which a 10 µL quench/detection solution (containing labeled tracer identified in Example 5 above), Tb-chelate-labeled anti-pSer133 antibody, and EDTA) was added. The plate was covered and incubated at room temperature for 2 hours. The plate was then read on a TECAN Ultra 384 fluorescence plate reader using a 340/35 nm excitation filter and 520/25 and 495/10 nm emission filters (Chroma Technology Corp.). Data was collected using 10 flashes per well with a 100 µs delay and 200 µs integration window. See FIG. 10A.

To assess assay robustness, a Z' value was determined from 48 20 µL wells containing Tb-chelate labeled anti-pSer133 antibody and labeled tracer (see above) in the presence (24 wells; "low signal" controls) or absence (24 wells, "high signal" controls) of 2.5 μM unlabeled tracer. The plate was covered and incubated for 2 hours at room temperature. The plate was then read on a TECAN Ultra 384 fluorescence plate reader using the parameters described above. The Z'-value was 0.92. See FIG. 10B.

Example 7 - Conversion of Nuclear Receptor FP Assay to Multilplex FP/TR-RET Assay

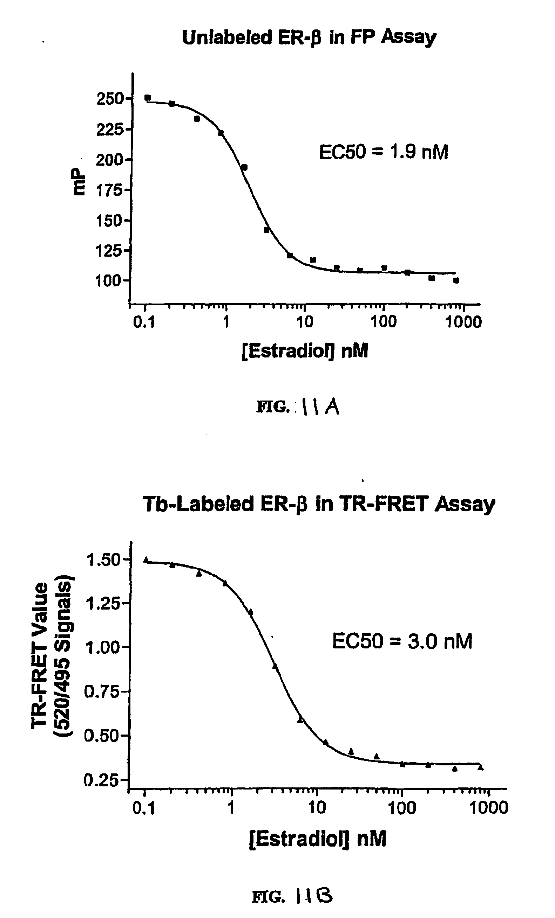

To demonstrate the generality of the ability to convert FP assays to FP/TR-RET assays using terbium chelates, an Estrogen Receptor P (ER-β) FP competition assay was converted by directly labeling the ER receptor with an amine-reactive terbium chelate; see Example 1 above. In the FP assay, displacement of a fluorescein-labeled tracer by a competitor causes a change in the observed polarization from high to low. In the TR-RET assay, the amount of labeled tracer bound to receptor is measured by RET between the terbium chelate on the receptor and the fluorescein on the tracer. In the absence of a competitor the RET signal is high, and as the competitor displaces the tracer this signal decreases. 12.5 nM unlabeled (see FIG. 11A) or Tb-chelate labeled ER-β protein (see FIG. 11B) were incubated with 1 nM labeled tracer (Fluormone ES2 (PanVera, Madison WI part#P2613)) and titrated with serial dilutions of unlabeled estradiol, a known ER-β ligand. Both FP and TR-RET assays showed similar EC50 values for the competition curve. In addition, the TR-RET assay offers the advantage that it could be re-formatted, with similar results expected, using limiting concentrations of receptor and excess concentrations of tracer.

Example 8 - Conversion of EGFR Kinase FP Assay to Multiplex FP/TR-RET Assay

The general method identified in Example 7 was used to screen for inhibitors of Epidermal Growth Factor Receptor (EGFR) Kinase (a protein tyrosine kinase) using the LOPAC (Sigma #LO1280) compound library. Hits identified in both readout modes were all seen to be true hits, whereas hits that showed discrepancy between readout modes were seen to be false. These results indicate that by multiplexing readout modes within an assay, one can significantly improve the integrity of the determined results.

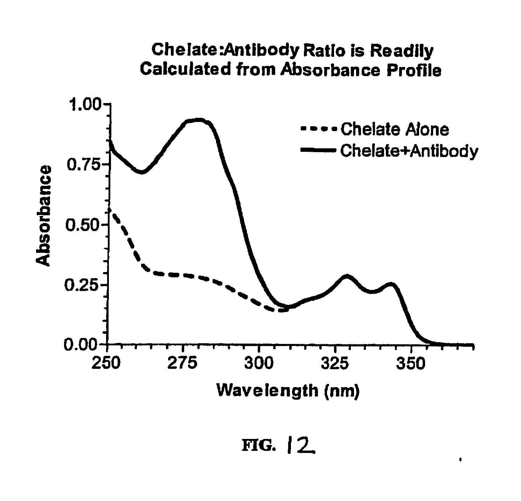

Anti p-Tyr antibody (anti-pY20 available from Zymed) was concentrated to 5 mg/mL in 100 mM sodium carbonate buffer, pH 9.5. CS124-DTPA-Phe-NCS*Tb (Tb-chelate) was added at a 5 to 40-fold molar excess relative to antibody, and the reaction incubated at room temperature for 4 hours with light vortexing every 30 minutes. After 4 hours, the antibody was dialyzed twice against PBS to remove unreacted and/or hydrolyzed chelate. The amount of chelate bound to the antibody was quantitated by the absorbance of the CS124 moiety at 343 nm ($E_{340}$ = 11,440 $M^{-1}cm^{-1}$), and the amount of antibody quantitated by its absorbance at 280 nm ($E_{280}$ = 210,000 $M^{-1}cm^{-1}$), correcting for the absorbance of the CS124 at 280 nM (1.1 x its absorbance at 343 nM). See FIG. 12.

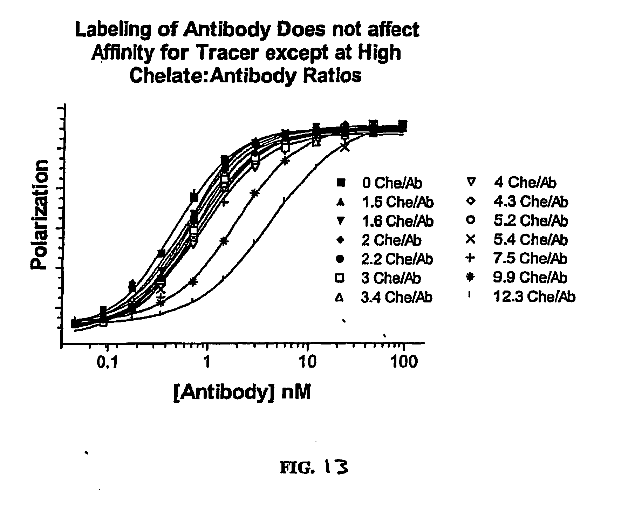

To determine whether labeling of the antibody affected its affinity for a fluorescein-labeled phosphopeptide tracer (see Example 2), binding curves were performed as previously described. At a labeling ratio of less than 9 chelates per antibody, the affinity for the tracer was seen to vary by less than 2-fold. See FIG. 13.

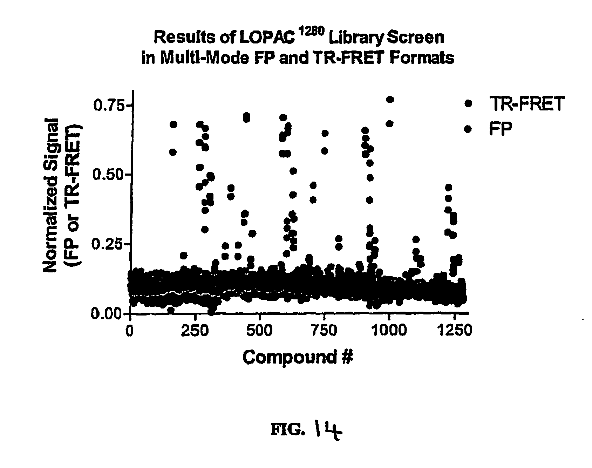

Epidermal Growth Factor Receptor (EGFR) Tyrosine Kinase (available from PanVera, Madison WI, #P2628) was screened for activity against the LOPAC$^{1280™}$ (Sigma #LO1280) library (containing 1280 compounds) in 10 μL reaction volume (20 μL detection volume) in Coming low-volume 384-well plates (part #3676). The kinase reaction was performed in the presence of 10 μM library compound under the following reaction conditions: 20 mM HEPES pH 7.5, 5 mM $MgCl_2$, 2mM $MnCl_2$, 0.05mM $Na_3VO_4$, 1mM DTT, 150 nM poly(GlyTyr) 4:1 poly-GT tyrosine kinase substrate, and 10 μM ATP using 0.1 unit of kinase per reaction. The reaction was allowed to proceed for 90 minutes at 30°C, after which a 10 μl solution of a 20 mM EDTA, 8 nM Tb-labeled anti-pTyr (anti pY72 antibody; see Examples 1 and 2) and 4 nM PTK labeled-tracer (see Example 2 above) in TR-RBT dilution buffer (PanVera, Madison WI part#PV3152) were added. The quenched reactions were then allowed to incubate for 1 hour at room temperature, after which they were read on a Tecan Ultra plate reader. Fluorescence Polarization was measured using a 485 nm excitation filter (20 nm bandpass) and 535 nm emission filters (25 nm bandpass). Time Resolved RET was measured using a 340 nm excitation filter (35 nm bandpass) and two emission filters; a 495nm with a 10 nm band pass for a reference peak and 520 nm with a 25nm band pass for signal change measurement, using a 200 μs integration window following a 100 μs post-flash delay. TR-RET filters were from Chroma Technology Corp. TR-RET values (ratios) were determined by dividing the intensity of the sample at 520 nm by the intensity of the sample at 495 nm. See FIG. 14.

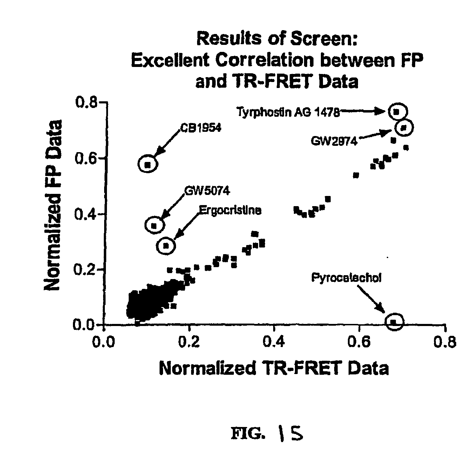

Data from the FP and TR-RET reads were normalized and plotted on orthogonal axes. See FIG. 15. The difference in the percent inhibition as determined by FP and TAR-RET was determined. Four compounds that fell outside of three standard deviations from this average (CB1954, GW5074, Ergocristine, Pyrocatechol) were identified for further analysis. In addition, two compounds (Tyrophostin AG 1478, GW2974) showing strong correlation between detection modes and strong inhibition were also selected for follow-up Profiling.

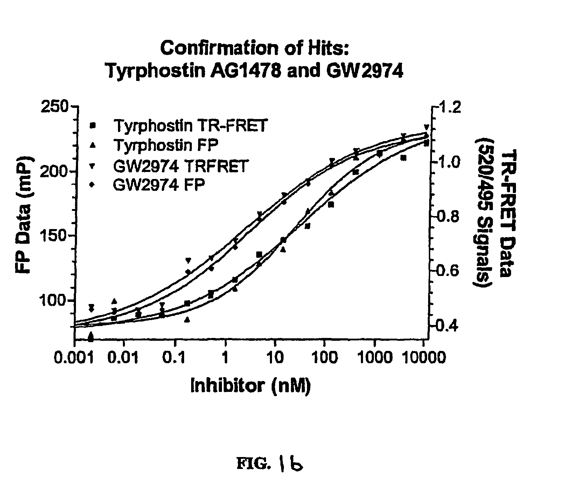

The two identified inhibitors (Tyrphostin AG1478 and GW2974, which are known inhibitors of EGFR kinase) were assayed in a series of 3-fold dilutions, and the four poorly-correlating compounds in a series of two-fold dilutions, against EGFR kinase under conditions as described in the library screen. Follow-up screening identified GW2974 as the more potent inhibitor, with an EC50 of about 10-folk less than that seen for AG1478. See FIG. 16.

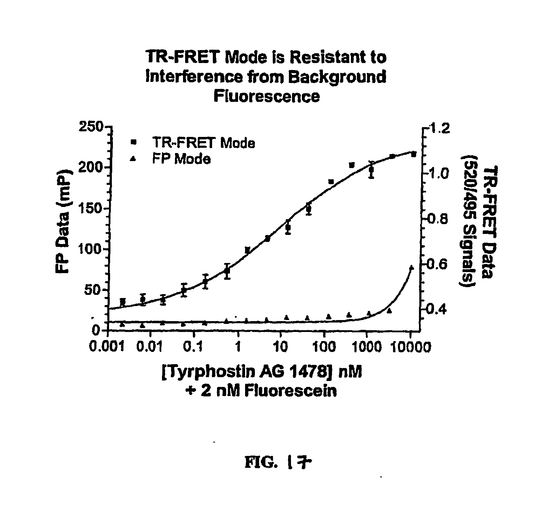

To demonstrate the ability of the TR-RET detection mode to identify true hits even in the presence of interfering background fluorescence (a useful criteria when identifying either hits that are intrinsically fluorescent, or when screening libraries of pooled compounds in which the presence of a fluorescent compound could mask the presence of a hit), the assay was performed against a dilution series of the inhibitor Typhostin AG1478 in the presence of 10 nM fluorescein. The TR-RBT data was seen to be impervious to the presence of the background fluorescence signal, whereas the FP data was severely compromised. See FIG. 17.

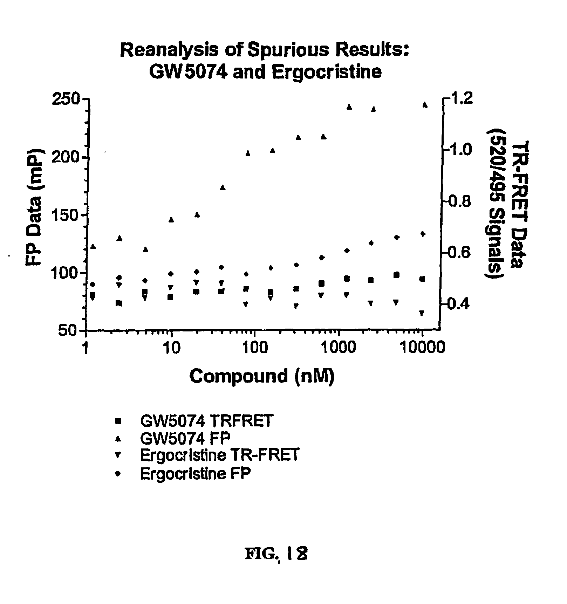

Two compounds that showed poor correlation between the FP and TR-RET detection modes, GW5074 and Ergocristine, were seen to precipitate, suggesting that the spurious signal in the FP detection mode was likely an artifact of light scatter. Because the signal due to scatter has a short lifetime, it does not affect the TR-RET reading mode. See FIG. 18.

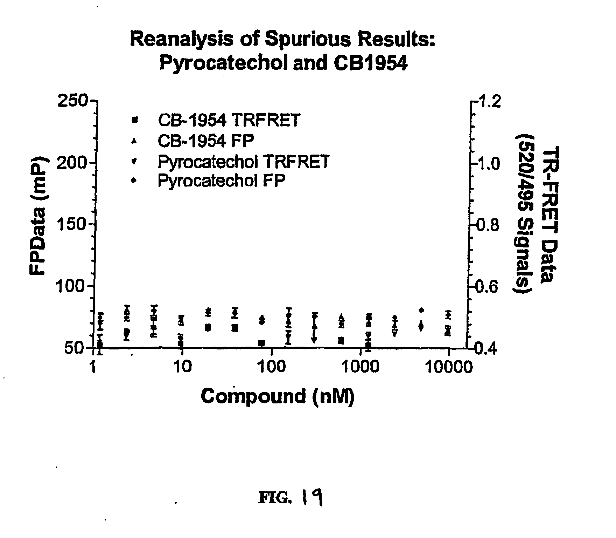

Two other compounds that showed poor correlation, CB-1954 and Pyrocatechol, were re-assayed and neither was seen to be an inhibitor. An examination of the screen showed that these compounds were in adjacent wells of the assay plate, suggesting a systematic error that led to the spurious results. See FIG. 19.

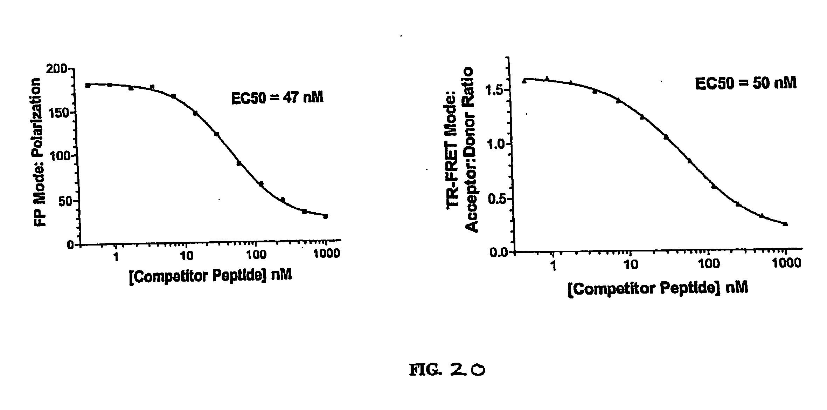

To assess the concentration of phosphorylated kinase product required to gave a detectable change in signal, a serial dilution of an unlabeled phosphorylated PTK tracer (as competitor product to tracer) was incubated with 4 nM Tb-chelate labeled anti-pTyr antibody and 2 nM fluorescein-labeled PTK tracer in TR-RET dilution buffer (see above). The plate was then read in both FP and TR-RET modes as described previously. The amount of competitor required for half-maximal signal change was seen to be nearly identical between assay modes, indicating that both assays bad similar sensitivities. See FIG 20.

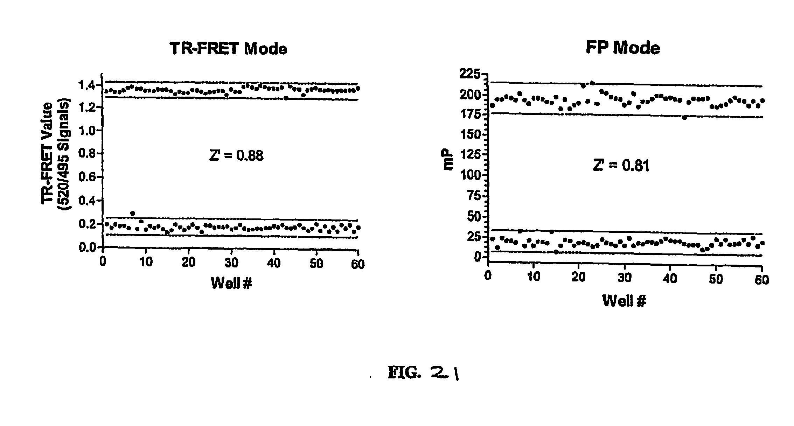

To assess assay robustness, 60 wells containing 4 nM Tb-chelate labeled anti-pTyr antibody and 2 nM fluorescein-labeled tracer (the "high value" controls), and 60 wells containing the same components in addition to 1 uM competitor peptide (the "low value" controls) were read in both FP and TR RET detection modes as described previously. Z' values were calculated according to Zhang et al., "A Simple Statistical Parameter for Use in Evaluation and Validation of High Throughput Screening Assays," Journal of Biomolecular Screening 4(2):67-73 (1999). The Z'-factor was seen to be > 0.8 for each assay mode. See FIG. 21.

Example 9- Detection of Histidine-tagged Proteins using Multiplex Modes

A multiplex system for the detection of His-tagged proteins or peptides was developed. The basis of the assay was a competition between a Histidine-tagged analyte protein and a tracer consisting of fluorescein linked to a hexahistidine peptide for a terbium-cholate labeled anti-His-tag antibody. In the absence of analyte protein or peptide, the fluorescein-labeled hexahistidine peptide associates with the anti-His-tag antibody, and this interaction can be detected by TR-RET or FP. In the presence of increasing amounts of analyte protein, this tracer-antibody interaction is disrupted and the TR-RET signal or fluorescence polarization of the tracer decreases. Fluorescein-His6 peptide (fluorescein-HHHHHH, the "luminescent tracer," SEQ ID NO:11) was synthesized by a commercial supplier (ResGen, Huntsville AL) and used as supplied. A commercial monoclonal antibody specific for the hexahistidine tag (Part MCA1396, Serotec, Raleigh, NC) was purchased and used as supplied with no additional purification. 0.25 mg antibody was concentrated in 100 mM sodium carbonate buffer, pH 9.5, to a final volume of 50 uL (5 mg / mL final concentration of antibody). To label the antibody, 30 ug of CS124-DTPA-Phe-NCS-Th (a 20-fold molar excess relative to antibody) was added and the reaction allowed to proceed at room temperature for 4 hours with light vortexing every 30 minutes. After 4 hours, the antibody was dialyzed twice versus PBS to remove unreacted and/or hydrolyzed chelate. The amount of chelate bound to the antibody was quantitated by the absorbance of the CS124 moiety at 343 nm ($E_{340}$= 11,440 $M^{-1}cm^{-1}$), and the amount of antibody quantitated by its absorbance at 280 nm ($E_{280}$ = 210,000 $M^{-1}cm^{-1}$), correcting for the absorbance of the CS124 at 280 nM (1.1 x its absorbance at 343 nM). From these measurements an average of 7.7 chelates per antibody was determined. The labeled antibody was seen to be stable for at least 6 months with no noticeable loss in performance.

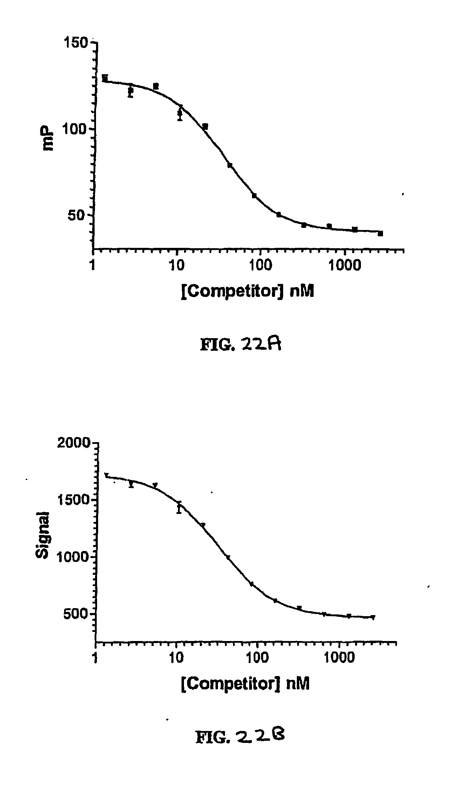

A competitive binding assay was performed with 20 nM antibody and 2 nM tracer, with titration of increasing amounts of His-tagged peptide (sequence: Biotin-KGGHHHHHH, source: ResGen; SEQ ID NO: 12) ranging from 3 uM to 1.5 nM in two-fold dilutions. The assay components were mixed in FP Dilution buffer (see above) and read after a 30 minute incubation on a Tecan Ultra plate reader using a 340 nm excitation filter (35 nm bandpass) and a 520 nm emission filter (25 nm bandpass). Data were collected using a 200 $\mu$s integration window after a 100 $\mu$s post-flash delay, with 10 flashes per well. The data are shown in FIG. 22A and B.

A number of embodiments of the invention have been described. Nevertheless, it will be understood that various modifications may be made without departing from the scope of the invention.

**EP 1 671 128 B1**

**Claims**

1. An article of manufacture comprising:

   a) packaging material;
   b) a first binding partner comprising a Tb(III) metal complex; and
   c) a second binding partner comprising a GFP, wherein said second binding partner specifically binds said first binding partner,

   and wherein one of the first and second binding partners has binding specificity for either the product or substrate of an enzymatic activity.

2. The article of claim 1 wherein the binding partners are an antibody or antibody fragment labelled with a Tb(III) chelate and a polypeptide binding partner for the antibody labelled with a GFP.

3. The article of claim 1 wherein the binding partners are: an antibody or antibody fragment and a composition having an epitope or epitope mimetic recognised by that antibody or antibody fragment.

4. The article of any preceding claim wherein the enzymatic activity is selected from the group consisting of kinase activity, phosphatase activity, glucuronidase activity, prenylation, glycosylation, methylation, demethylation, acylation, acetylation, ubiquitination, sulfation, proteolysis, nuclease activity, nucleic acid polymerase activity, nucleic acid reverse transcriptase activity, nucleotidyl transferase activity, and polynucleotide translation activity.

5. The article of claim 4 wherein the enzymatic activity is kinase activity or phosphatase activity.

6. The article of claim 5 wherein the enzymatic activity is kinase activity.

7. The article of claim 1 wherein one of the binding partners includes a phosphorylated amino acid.

8. The article of claim 7 wherein the phosphorylated amino acid is a phosphorylated tyrosine, a phosphorylated serine, or a phosphorylated threonine.

9. The article of claim 1 wherein the enzymatic activity is tyrosine kinase activity and one of the binding partners is an antibody with specificity for a phosphorylated tyrosine as compared to an unmodified tyrosine, the other of the binding partners including the epitope recognised by the antibody or a mimetic of such epitope.

10. The article of claim 9 wherein the antibody is a monoclonal antibody, or is a single chain Fv antibody fragment, Fab fragment or $F(ab)_2$ fragment.

11. The article of any preceding claim wherein the Tb(III) containing complex has one of the following structures:

$$-L_n-A-S_n-C_M,$$

or

$$-L_nC_M-S_n-A,$$

   wherein A represents an organic antenna moiety;
   L represents a linker;
   S represents a spacer;
   n is 0 or 1;
   C represents a metal chelating moiety; and
   M represents a Tb(III) metal ion coordinated to C.

12. The article of claim 11 wherein the organic antenna moiety is a polynuclear heterocyclic aromatic compound.

13. The article of claim 11 or claim 12 where L is -NH-CO-NH-;

22

-CO-(CH$_2$)$_n$-NH- where n=1 to 10; -NH-Ph-; -NH-(CH$_2$)$_n$- , wherein n=1 to 10; -CO-NH-; -(CH$_2$)$_n$-NH- where n=1 to 10; -CO-(CH$_2$)$_n$-NH-, where n=1 to 10; or -CS-NH-.

14. The article of any of claims 11 to 13 wherein S is -NH-CO-NH-;

-CO-(CH$_2$)$_n$-NH- where n=1 to 10; -NH-Ph-; -NH-(CH$_2$)$_n$-, wherein n=1 to 10; -CO-NH-; -(CH$_2$)$_n$-NH- where n=1 to 10; -CO-(CH$_2$)$_n$-NH-, where n=1 to 10; or -CS-NH-.

15. The article of any preceding claim wherein the GFP is a mutant GFP.

16. The article of claim 15 wherein the mutant GFP is a mutant of GFP from *Aequorea victoria.*

17. The article of claim 15 wherein the mutant GFP is a GFP of Table 7 of US-A-6410255, namely a mutation listed in the following Table:

| *Mutations* | *Common Name* |
|---|---|
| S65 type | |
| S65T, S72A, N149K, M153T, I167T | Emerald |
| F64L, S65T, V163A | |
| F64L, S65T (EGFP) | EGFP |
| S65T | |
| Y66H type | |
| F64L, Y66H, Y145F, V163A | P4-3E |
| F64L, Y66H, Y145F | |
| Y66H, Y145F | P4-3 |
| Y66H | BFP |
| Y66W type | |
| S65A, Y66W, S72A, N1461, M153T, | W1C |
| V 163A | |
| F64L, S65T, Y66W, N1461, M153T, | W1B |
| V 163A | |
| Y66W, N146I, M153T, V163A | hW7 |
| Y66W | |
| T203Y type | |
| S65G, S72A, K79R, T203Y | Topaz |
| S65G, V68L, S72A, T203Y | 10C |
| S65G, V68L, Q69K, S72A, T203Y | h10C+ |
| S65G, S72A, T203H | |
| S65G, S72A, T203F | |

(continued)

| T203I type | |
|---|---|
| T203I, S72A, Y145F | Sapphire |
| T203I, T202F | H9 |

18. A method for forming a test sample, comprising

contacting an enzyme with a substrate for the enzyme under conditions effective for an enzymatic activity of the enzyme to form a product from the substrate, the contacting being carried out in the presence of a potential modulator of the enzymatic activity, and
forming a test sample by either

i) contacting the mixture of the enzyme, substrate and potential modulator with (a) a binding partner having specificity for either the substrate or a product of enzymatic activity on the substrate, and (b) a luminescent tracer capable of binding with the binding partner, wherein one of the binding partner and the tracer includes a luminescent Tb(III)-containing metal complex whilst the other includes a GFP; or
ii) contacting the mixture of the enzyme, substrate and potential modulator with (a) a binding partner having specificity for either the substrate or a product of enzymatic activity on the substrate, and (b) a tracer capable of binding with the binding partner, wherein one of the binding partner and the substrate includes a luminescent Tb(III)-containing metal complex whilst the other includes a GFP.

19. The method of claim 18 which further comprises exposing the test sample to polarised light at an appropriate wavelength and measuring the polarisation of fluorescent emission from the test sample.

20. The method of claim 19 which further comprises exposing the test sample to light and measuring fluorescence emission from the test sample.

21. The method of any of claims 18 to 20 wherein the tracer includes the purported epitope recognised by a first binding partner which is an antibody.

22. The method of claim 21 wherein the binding partner is an antibody, e.g. a monoclonal antibody or an antibody fragment, with specificity for a phosphorylated tyrosine as compared to an unmodified tyrosine.

23. The method of any of claims 18 to 22 wherein the enzyme has an activity listed in claim 4, claim 5 or claim 6

24. The method of claim 23 wherein the enzyme has kinase activity.

25. Use of an article of any of claims 1 to 17 in performing monitoring by one or more fluorescent techniques an interaction of first and second binding partners, wherein the first binding partner comprises a Tb(III)-containing metal complex and the second binding partner comprises a GFP, and one of the first and second binding partners has binding specificity for either the product or substrate of an enzymatic activity.

26. Use of claim 25 wherein the first binding partner has binding specificity for the product of the enzymatic activity.

27. Use of claim 25 wherein the first binding partner has binding specificity for the substrate of the enzymatic activity.

28. Use of claim 26 wherein the first binding partner is an antibody or antibody fragment specific for a particular post-translational modification of a protein.

29. Use of claim 28 wherein the post-translational modification is phosphorylation or de-phosphorylat ion.

**Patentansprüche**

1. Ein Erzeugnis, das folgendes umfasst:

24

a) Verpackungsmaterial;

b) einen ersten Bindungspartner, der einen Tb(III)-Metallkomplex umfasst; und

c) einen zweiten Bindungspartner, der ein GFP umfasst, wobei der zweite Bindungspartner spezifisch an den ersten Bindungspartner bindet,

und wobei einer der ersten und zweiten Bindungspartner eine Bindungsspezifität für entweder das Produkt oder Substrat einer enzymatischen Aktivität aufweist.

2. Das Erzeugnis nach Anspruch 1, wobei die Bindungspartner ein Antikörper oder Antikörperfragment, das mit einem Tb(III)-Chelat markiert ist, und ein Polypeptid-Bindungspartner für den mit einem GFP markierten Antikörper sind.

3. Das Erzeugnis nach Anspruch 1, wobei die Bindungspartner folgende sind: ein Antikörper oder Antikörperfragment und eine Zusammensetzung, die ein Epitop oder Epitop-Mimetikum aufweist, das von dem Antikörper oder Antikörperfragment erkannt wird.

4. Das Erzeugnis nach einem der vorstehenden Ansprüche, wobei die enzymatische Aktivität aus der Gruppe ausgewählt wird, die aus folgendem besteht: Kinase-Aktivität, Phosphatase-Aktivität, Glucuronidase-Aktivität, Prenylierung, Glycosylierung, Methylierung, Demethylierung, Acylierung, Acetylierung, Ubiquitinierung, Sulfatierung, Proteolyse, Nuklease-Aktivität, Nukleinsäurepolymerase-Aktivität, Nukleinsäuren-Reverse-Transkriptase-Aktivität, Nukleotidyltransferase-Aktivität und Polynukleotidtranslation-Aktivität.

5. Das Erzeugnis nach Anspruch 4, wobei die enzymatische Aktivität Kinase-Aktivität oder Phosphatase-Aktivität ist.

6. Das Erzeugnis nach Anspruch 5, wobei die enzymatische Aktivität Kinase-Aktivität ist.

7. Das Erzeugnis nach Anspruch 1, wobei einer der Bindungspartner eine phosphorylierte Aminosäure einschließt.

8. Das Erzeugnis nach Anspruch 7, wobei die phosphorylierte Aminosäure ein phosphoryliertes Tyrosin, ein phosphoryliertes Serin oder ein phosphoryliertes Threonin ist.

9. Das Erzeugnis nach Anspruch 1, wobei die enzymatische Aktivität Tyrosinkinase-Aktivität ist und einer der Bindungspartner ein Antikörper mit Spezifität für ein phosphoryliertes Tyrosin im Vergleich zu einem nicht modifizierten Tyrosin ist, und der andere Bindungspartner das Epitop, das von dem Antikörper erkannt wird, oder ein Mimetikum eines solchen Epitops einschließt.

10. Das Erzeugnis nach Anspruch 9, wobei der Antikörper ein monoklonaler Antikörper ist oder ein Einzelketten-Fv-Antikörperfragment, Fab-Fragment oder $F(ab)_2$-Fragment ist.

11. Das Erzeugnis nach einem der vorstehenden Ansprüche, wobei der Tb(III)-haltige Komplex eine der folgenden Strukturen aufweist:

$$-L_n-A-S_n-C_M,$$

oder

$$-L_n-C_M-S_n-A,$$

worin A eine organische Antennengruppe darstellt;

L einen Linker darstellt;

S einen Spacer darstellt;

n 0 oder 1 ist;

C eine metallchelatisierende Gruppe darstellt; und

M ein an C koordiniertes Tb(III)-Metallion darstellt.

12. Das Erzeugnis nach Anspruch 11, wobei die organische Antennengruppe eine mehrkernige heterocyclische aromatische Verbindung ist.

13. Das Erzeugnis nach Anspruch 11 oder Anspruch 12, wobei folgendes gilt: L ist -NH-CO-NH-; -CO-$(CH_2)_n$-NH- mit

n = 1 bis 10; -NH-Ph-; -NH-(CH$_2$)$_n$-mit n = 1 bis 10; -CO-NH-; -(CH$_2$)$_n$-NH- mit n = 1 bis 10; -CO-(CH$_2$)$_n$-NH- mit n = 1 bis 10; oder -CS-NH-.

**14.** Das Erzeugnis nach einem der Ansprüche 11 bis 13, wobei folgendes gilt: S ist -NH-CO-NH-; -CO-(CH$_2$)$_n$-NH- mit n = 1 bis 10; -NH-Ph-; -NH-(CH$_2$)$_n$-mit n = 1 bis 10; -CO-NH-; -(CH$_2$)$_n$-NH- mit n = 1 bis 10; -CO-(CH$_2$)$_n$-NH- mit n = 1 bis 10; oder -CS-NH-.

**15.** Das Erzeugnis nach einem der vorstehenden Ansprüche, wobei das GFP ein Mutanten-GFP ist.

**16.** Das Erzeugnis nach Anspruch 15, wobei das Mutanten-GFP ein Mutanten-GFP aus Aequorea victoria ist.

**17.** Das Erzeugnis nach Anspruch 15, wobei das Mutanten-GFP ein GFP aus Tabelle 7 der US-A-6410255 ist, nämlich eine in der folgenden Tabelle aufgeführte Mutation:

| Mutationen | Gebräuchliche Bezeichnung |
|---|---|
| S65-Typ | |
| S65T, S72A, N149K, M153T, I167T | Smaragd |
| F64L,S65T,V163A | |
| F64L, S65T (EGFP) | (EGFP) |
| S65T | |
| Y66H-Typ | |
| F64L, Y66H, Y145F, V163A | P4-3E |
| F64L, Y66H, Y145F | |
| Y66H, Y145F | P4-3 |
| Y66H | BFP |
| Y66W-Typ | |
| S65A, Y66W, S72A, N1461, M153T, V163A | W1C |
| F64L, S65T, Y66W, N1461, M153T, V163A | W1B |
| Y66W, N146I, M153T, V163A | hW7 |
| Y66W | |
| T203Y-Typ | |
| S65G, S72A, K79R, T203Y | Topas |
| S65G, V68L, S72A, T203Y | 10C |
| S65G, V68L, Q69K, S72A, T203Y | h10C+ |
| S65G, S72A, T203H | |
| S65G, S72A, T203F | |
| T203I-Typ | |
| T203I, S72A, Y145F | Saphir H9 |
| T2031, T202F | |

**18.** Ein Verfahren zur Bildung einer Testprobe, das folgendes umfasst:

Kontaktieren eines Enzyms mit einem Substrat für das Enzym unter Bedingungen, die für eine enzymatische Aktivität des Enzyms wirksam sind, um ein Produkt aus dem Substrat zu bilden, wobei das Kontaktieren in Gegenwart eines potenziellen Modulators der enzymatischen Aktivität durchgeführt wird, und

**EP 1 671 128 B1**

Bilden einer Testprobe entweder über

i) Kontaktieren des Gemischs aus dem Enzym, Substrat und potenziellem Modulator mit (a) einem Bindungspartner, der eine Spezifität für entweder das Substrat oder einem Produkt einer enzymatischen Aktivität an dem Substrat aufweist, und (b) einem Lumineszenzmarker, der zur Bindung mit dem Bindungspartner in der Lage ist, wobei eines aus dem Bindungspartner und dem Marker einen lumineszierenden Tb(III)-haltigen Metallkomplex einschließt, während das andere ein GFP einschließt; oder
ii) Kontaktieren des Gemischs aus dem Enzym, Substrat und potenziellem Modulator mit (a) einem Bindungspartner, der eine Spezifität für entweder das Substrat oder einem Produkt einer enzymatischen Aktivität an dem Substrat aufweist, und (b) einem Marker, der zur Bindung mit dem Bindungspartner in der Lage ist, wobei eines aus dem Bindungspartner und dem Substrat einen lumineszierenden Tb(III)-haltigen Metallkomplex einschließt, während das andere ein GFP einschließt

19. Das Verfahren nach Anspruch 18, das ferner das Aussetzen der Testprobe gegenüber polarisiertem Licht bei einer zweckmäßigen Wellenlänge und das Messen der Polarisation der Fluoreszenzemission aus der Testprobe umfasst.

20. Das Verfahren nach Anspruch 19, das ferner das Aussetzen der Testprobe gegenüber Licht und das Messen der Fluoreszenzemission aus der Testprobe umfasst.

21. Das Verfahren nach einem der Ansprüche 18 bis 20, wobei der Marker das angebliche Epitop einschließt, das von einem ersten Bindungspartner erkannt wird, der ein Antikörper ist.

22. Das Verfahren nach Anspruch 21, wobei der Bindungspartner ein Antikörper, z. B. ein monoklonaler Antikörper oder ein Antikörperfragment, mit Spezifität für ein phosphoryliertes Tyrosin im Vergleich zu einem nicht modifizierten Tyrosin ist.

23. Das Verfahren nach einem der Ansprüche 18 bis 22, wobei das Enzym eine Aktivität aufweist, die in Anspruch 4, Anspruch 5 oder Anspruch 6 aufgeführt ist.

24. Das Verfahren nach Anspruch 23, wobei das Enzym Kinase-Aktivität aufweist.

25. Verwendung eines Erzeugnisses nach einem der Ansprüche 1 bis 17 bei der Durchführung der Verfolgung über ein oder mehr als ein Fluoreszenzverfahren einer Wechselwirkung von ersten und zweiten Bindungspartnern, wobei der erste Bindungspartner einen Tb(III)-haltigen Metallkomplex umfasst und der zweite Bindungspartner ein GFP umfasst und einer der ersten und zweiten Bindungspartner eine Bindungsspezifität für entweder das Produkt oder Substrat einer enzymatischen Aktivität aufweist.

26. Verwendung nach Anspruch 25, wobei der erste Bindungspartner eine Bindungsspezifität für das Produkt der enzymatischen Aktivität aufweist.

27. Verwendung nach Anspruch 25, wobei der erste Bindungspartner eine Bindungsspezifität für das Substrat der enzymatischen Aktivität aufweist.

28. Verwendung nach Anspruch 26, wobei der erste Bindungspartner ein Antikörper oder Antikörperfragment ist, das für eine bestimmte post-translationale Modifikation eines Proteins spezifisch ist.

29. Verwendung nach Anspruch 28, wobei die post-translationale Modifikation eine Phosphorylierung oder De-phosphorylierung ist.

**Revendications**

1. Article de fabrication comprenant :

   a) une matière d'emballage ;
   b) un premier partenaire de liaison comprenant un complexe métallique de Tb(III) ; et
   c) un second partenaire de liaison comprenant une GFP, ledit second partenaire de liaison se liant spécifiquement audit premier partenaire de liaison,

et dans lequel l'un parmi des premier et second partenaires de liaison a une spécificité de liaison soit pour le produit soit pour le substrat d'une activité enzymatique.

2. Article selon la revendication 1, dans lequel les partenaires de liaison sont un anticorps ou fragment d'anticorps marqué par un chélate de Tb(III) et un partenaire de liaison polypeptidique pour l'anticorps marqué par une GFP.

3. Article selon la revendication 1, dans lequel les partenaires de liaison sont : un anticorps ou fragment d'anticorps et une composition ayant un épitope ou mimétique d'épitope reconnu par cet anticorps ou fragment d'anticorps.

4. Article selon l'une quelconque des revendications précédentes, dans lequel l'activité enzymatique est choisie dans le groupe consistant en une activité kinase, une activité phosphatase, une activité glucuronidase, une prénylation, une glycosylation, une méthylation, une déméthylation, une acylation, une acétylation, une ubiquitination, une sulfatation, une protéolyse, une activité nucléase, une activité acide nucléique polymérase, une activité acide nucléique transcriptase inverse, une activité nucléotidyl transférase et une activité de traduction de polynucléotide.

5. Article selon la revendication 4, dans lequel l'activité enzymatique est une activité kinase ou une activité phosphatase.

6. Article selon la revendication 5, dans lequel l'activité enzymatique est une activité kinase.

7. Article selon la revendication 1, dans lequel l'un des partenaires de liaison comprend un acide aminé phosphorylé.

8. Article selon la revendication 7, dans lequel l'acide aminé phosphorylé est une tyrosine phosphorylée, une sérine phosphorylée ou une thréonine phosphorylée.

9. Article selon la revendication 1, dans lequel l'activité enzymatique est une activité tyrosine kinase et l'un des partenaires de liaison est un anticorps avec une spécificité pour une tyrosine phosphorylée par comparaison avec une tyrosine non modifiée, l'autre partenaire de liaison comprenant l'épitope reconnu par l'anticorps ou un mimétique d'un tel épitope.

10. Article selon la revendication 9, dans lequel l'anticorps est un anticorps monoclonal, ou est un fragment d'anticorps Fv à chaîne unique, un fragment Fab ou un fragment $F(ab)_2$.

11. Article selon l'une quelconque des revendications précédentes, dans lequel le complexe contenant du Tb(III) a l'une des structures suivante

$$-L_n-A-S_n-C_M,$$

ou

$$-L_n-C_M-S_n-A,$$

où A représente une fraction antenne organique ;
L représente un lieur,
S représente un espaceur,
n est 0 ou 1 ;
C représente une fraction chélatant le métal ; et
M représente un ion métallique Tb(III) coordonné à C.

12. Article selon la revendication 11, dans lequel la fraction antenne organique est un composé aromatique hétérocyclique polynucléaire.

13. Article selon la revendication 11 ou la revendication 12, dans lequel L est -NH-CO-NH- ;

-CO-$(CH_2)_n$-NH- où n = 1 à 10 ; -NH-Ph- ; NH-$(CH_2)_n$-, où n = 1 à 10 ; -CO-NH-, -$(CH_2)_n$-NH- où n = 1 à 10 ; -CO-$(CH_2)_n$-NH-, où n = 1 à 10 ; ou -CS-NH-.

14. Article selon l'une quelconque des revendications 11 à 13, dans lequel S est -NH-CO-NH- ; -CO-$(CH_2)_n$-NH- où n = 1 à 10 ; -NH-Ph- ; -NH-$(CH_2)_n$-, où n = 1 à 10 ; -CO-NH- ; -$(CH_2)_n$-NH- où n = 1 à 10 ; -CO-$(CH_2)_n$-NH, où n = 1 à 10 ; ou -CS-NH.

**15.** Article l'une quelconque des revendications précédentes, dans lequel la GFP est une GFP mutante.

**16.** Article selon la revendication 15, dans lequel la GFP mutante est un mutant de GFP d'*Aequorea victoria.*

**17.** Article selon la revendication 15, dans lequel la GFP mutante est une GFP du Tableau 7 de US-A-6410255, à savoir une mutation énumérée dans le Tableau suivant :

| Mutations | Dénomination Commune |
|---|---|
| type S65 | |
| S65T, S72A, N149K, M153T, I167T | Emeraude |
| F64L, S65T, V163A | |
| F64L, S65T (EGFP) | EGFP |
| S65T | |
| type Y66H | |
| F64L, Y66H, Y145F, V163A | P4-3E |
| F64L, Y66H, Y145F | |
| Y66H, Y145F | P4-3 |
| Y66H | BFP |
| type Y66W | |
| S65A, Y66W, S72A, N146I, M153T, V163A | W1C |
| F64L, S65T, Y66W, N146I, M153T, V163A | W1B |
| Y66W, N146I, M153T, V163A | hW7 |
| Y66W | |
| type T203Y | |
| S65G, S72A, K79R, T203Y | Topaze |
| S65G, V68L, S72A, T203Y | 10C |
| S65G, V68L, Q69K, S72A, T203Y | h10C+ |
| S65G, S72A, T203H | |
| S65G, S72A, T203F | |
| type T203I | |
| T203I, S72A, Y145F | Saphir |
| T203I, T202F | H9 |

**18.** Procédé pour former un échantillon de test, comprenant
la mise en contact d'une enzyme avec un substrat pour l'enzyme dans des conditions efficaces pour une activité enzymatique de l'enzyme afin de former un produit à partir du substrat, la mise en contact étant conduite en présence d'un modulateur potentiel de l'activité enzymatique, et la formation d'un échantillon de test par soit :

i) la mise en contact du mélange de l'enzyme, du substrat et du modulateur potentiel avec (a) un partenaire de liaison ayant une spécificité pour soit le substrat soit un produit d'une activité enzymatique sur le substrat, et (b) un traceur luminescent capable de se lier avec le partenaire de liaison, l'un du partenaire de liaison et du traceur comprenant un complexe métallique contenant du Tb(III) luminescent tandis que l'autre comprend une GFP ; soit

ii) la mise en contact du mélange de l'enzyme, du substrat et du modulateur potentiel avec (a) un partenaire de liaison ayant une spécificité pour soit le substrat soit un produit d'une activité enzymatique sur le substrat, et (b) un traceur capable de se lier avec le partenaire de liaison, l'un du partenaire de liaison et du substrat comprenant un complexe métallique contenant du Tb(III) luminescent tandis que l'autre comprend une GFP.

19. Procédé selon la revendication 18, qui comprend de plus l'exposition de l'échantillon de test à une lumière polarisée à une longueur d'onde appropriée et la mesure de la polarisation d'émission fluorescente à partir de l'échantillon de test.

20. Procédé selon la revendication 19, qui comprend de plus l'exposition de l'échantillon de test à la lumière et la mesure de l'émission de fluorescence à partir de l'échantillon de test.

21. Procédé selon l'une quelconque des revendications 18 à 20, dans lequel le traceur comprend l'épitope présumé reconnu par un premier partenaire de liaison qui est un anticorps.

22. Procédé selon la revendication 21, dans lequel le partenaire de liaison est un anticorps, par exemple un anticorps monoclonal ou un fragment d'anticorps, avec une spécificité pour une tyrosine phosphorylée par comparaison avec une tyrosine non modifiée.

23. Procédé selon l'une quelconque des revendications 18 à 22, dans lequel l'enzyme a une activité énumérée dans la revendication 4, la revendication 5 ou la revendication 6.

24. Procédé selon la revendication 23, dans lequel l'enzyme a une activité kinase.

25. Utilisation d'un article selon l'une quelconque des revendications 1 à 17 dans la conduite d'une surveillance par une ou plusieurs techniques fluorescentes d'une interaction des premier et second partenaires de liaison, le premier partenaire de liaison comprenant un complexe métallique contenant du Tb(III), et le second partenaire de liaison comprenant une GFP, et l'un parmi les premier et second partenaires de liaison ayant une spécificité de liaison soit pour le produit soit pour le substrat d'une activité enzymatique.

26. Utilisation selon la revendication 25, dans laquelle le premier partenaire de liaison a une spécificité de liaison pour le produit de l'activité enzymatique.

27. Utilisation selon la revendication 25, dans laquelle le premier partenaire de liaison a une spécificité de liaison pour le substrat de l'activité enzymatique.

28. Utilisation selon la revendication 26, dans laquelle le premier partenaire de liaison est un anticorps ou fragment d'anticorps spécifique pour une modification post-traductionnelle particulière d'une protéine.

29. Utilisation selon la revendication 28, dans laquelle la modification post-traductionnelle est une phosphorylation ou une déphosphorylation.

**Bound Tracer (High TR-FRET)**

**Bound Tracer (High FP)**

FIG. 1

CS124-DTPA-Phe-NCS * Tb

FIG. 2

CS124-DTPA-EMCH * Tb

FIG. 3

FIG. 4

FIG. 5.

**PTK Direct Binding Assay:**
Tb-PY72 Antibody Titrated
Against 1 nM PTKTracer

FIG. 6

**PTK Competition Assay:**
10nM Tb-PY72 Ab, 1nM PTK Green Tracer
Titrated with Phosphopeptide Competitor

FIG. 7

FIG. 8A

FIG. 8B

FP Results: Anti-pCREB Antibody
with Four Different Tracers

FIG. 9A

TR-FRET Results: Tb-Labeled Anti pCREB
with Four Different Tracers

FIG. 9

## PKA Enzyme Titration

**FIG. 10A**

**FIG. 10B**

## Unlabeled ER-β in FP Assay

EC50 = 1.9 nM

FIG. 11A

## Tb-Labeled ER-β in TR-FRET Assay

EC50 = 3.0 nM

FIG. 11B

Chelate:Antibody Ratio is Readily
Calculated from Absorbance Profile

FIG. 12

Labeling of Antibody Does not affect
Affinity for Tracer except at High
Chelate:Antibody Ratios

FIG. 13

Results of LOPAC[1280] Library Screen
in Multi-Mode FP and TR-FRET Formats

FIG. 14

**Results of Screen:
Excellent Correlation between FP
and TR-FRET Data**

FIG. 15

Confirmation of Hits:
Tyrphostin AG1478 and GW2974

FIG. 1b

**TR-FRET Mode is Resistant to Interference from Background Fluorescence**

FIG. 17

Reanalysis of Spurious Results:
GW5074 and Ergocristine

- ■ GW5074 TRFRET
- ▲ GW5074 FP
- ▼ Ergocristine TR-FRET
- ◆ Ergocristine FP

FIG. 18

**Reanalysis of Spurious Results: Pyrocatechol and CB1954**

- CB-1954 TRFRET
- CB-1954 FP
- Pyrocatechol TRFRET
- Pyrocatechol FP

FIG. 19

FIG. 20

FIG. 21

EP 1 671 128 B1

FIG. 22A

FIG. 22B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4946778 A
- WO 0301115 A
- WO 6410255 A
- WO 9623526 A
- WO 0109188 A
- WO 0108712 A
- WO 03011115 A
- US 5639615 A
- US 5656433 A
- US 5622821 A
- US 5571897 A
- US 5534622 A
- US 5220012 A
- US 5162508 A
- US 4927923 A
- US 6410255 B
- US 5625048 A, Tsien
- US 5777079 A, Tsien
- US 5804387 A, Cormack
- US 6511815 B
- US 5445935 B
- US 4822733 A
- US 5527684 A
- US 6352672 B

### Non-patent literature cited in the description

- **Hemmila I et al.** *Drug Discovery Today,* 1997, vol. 2 (9), 373-381
- **Zhang Y et al.** *J. Assoc. Lab. Autom.,* 2003, vol. 8 (2), 71-73
- **Zaman G J R et al.** *Combinat. Chem. High Throughput Screen,* 2003, vol. 6 (4), 3113-320
- **Selvin PR.** *Annu. Rev. Biophs. Biomol. Struct.,* 2002, vol. 31, 275-302
- **Lakowicz, J.R.** Topics in Fluorescence Spectroscopy. Plenum Press, 1991
- **Lakowicz, J. R.** Emerging applications of fluorescence spectroscopy to cellular imaging: lifetime imaging, metal-ligand probes, multi photon excitation and light quenching. *Scanning Microsc.,* 1996, vol. 10, 213-24
- **Sambrook et al.** Molecular Cloning: A Laboratory Manual. Cold Spring Harbor Laboratory Press, 1989
- Cells: A Laboratory Manual. Cold Spring Harbor Laboratory Press, 1998
- **Snyder ; Love.** Optical Waveguide Theory. Chapman & Hall
- **Lakowicz, J.R.** Topics in Fluorescence Spectroscopy. Plenum Press, 1991, vol. 1-3
- Resonance Energy Transfer Microscopy, in Fluorescence Microscopy of Living Cells in Culture. **Herman, B.** Part B, Methods in Cell Biology. Academic Press, 1989, vol. 30, 219-243
- **Turro, N.J.** Modem Molecular Photochemistry. Benjamin/Cummings Publishing Col, Inc, 1978, 296-361
- **Bernard Valeur.** Molecular Fluorescence: Principles and Applications. Wiley VCH, 2002
- **Berlman, I.B.** Energy transfer parameters of aromatic compounds. Academic Press, 1973
- *Molecular Probes Catalog,* 2003
- **Tsien et al.** Handbook of Biological Confocal Microscopy. 1990, 169-178
- The American Chemical Society Style Guide. American Chemical Society, 1997
- Guidelines for Authors. 2001
- *J. Org. Chem.,* 2001, vol. 66 (1), 24A
- A Short Guide to Abbreviations and Their Use in Peptide Science. J. Peptide. Sci. 1999, vol. 5, 465-471
- **Spatola, A.F.** Chemistry and Biochemistry of Amino Acids, Peptides and Proteins. Marcel Dekker, 1983, 267
- **Kohler et al.** *Nature,* 1975, vol. 256, 495-497
- **Kosbor et al.** *Immunology Today,* 1983, vol. 4, 72
- **Cote et al.** *Proc. Natl. Acad. Sci. USA,* 1983, vol. 80, 2026-2030
- **Cole et al.** Monoclonal Antibodies and Cancer Therapy. Alan R. Liss, Inc, 1983, 77-96
- **Huse et al.** *Science,* 1989, vol. 246, 1275-1281
- Short Protocols in Molecular Biology. Green Publishing Associates and John Wiley & Sons, 1992
- **Flohe et al.** *Biochim. Biophys. Acta.,* 1970, vol. 220, 469-476
- **Tilgmann et al.** *FEBS,* 1990, vol. 264, 95-99
- PCR Primer: A Laboratory Manual. Cold Spring Harbor Laboratory Press, 1995

- **Lewis.** *Genetic Engineering News,* 1992, vol. 12 (9), 1
- **Guatelli et al.** *Proc. Natl. Acad. Sci. USA,* 1990, vol. 87, 1874-1878
- **Weiss.** *Science,* 1991, vol. 254, 1292
- **Green.** Fluorescent Proteins. Academic Press, 19-47
- Polarization of the fluorescence of solutions. **Weber.** Fluorescence and Phosphorescence Analysis. Interscience Publishers, 1966, 217-240
- **Zhang et al.** A Simple Statistical Parameter for Use in Evaluation and Validation of High Throughput Screening Assays. *Journal of Biomolecular Screening,* 1999, vol. 4 (2), 67-73